(19) Europäisches Patentamt
European Patent Office
Office européen des brevets

(11) Veröffentlichungsnummer : **0 102 591**
**B1**

(12) **EUROPÄISCHE PATENTSCHRIFT**

(45) Veröffentlichungstag der Patentschrift : **30.12.86**

(21) Anmeldenummer : **83108365.4**

(22) Anmeldetag : **25.08.83**

(51) Int. Cl.[4] : **C 07 D233/60**, C 07 D249/08, C 07 D405/06, A 01 N 43/653, A 01 N 43/50// C07D317/26

# (54) Substituierte Azolylether-ketone und -carbinole.

(30) Priorität : **07.09.82 DE 3233173**

(43) Veröffentlichungstag der Anmeldung : **14.03.84 Patentblatt 84/11**

(45) Bekanntmachung des Hinweises auf die Patenterteilung : **30.12.86 Patentblatt 86/52**

(84) Benannte Vertragsstaaten :
**AT BE CH DE FR GB IT LI NL SE**

(56) Entgegenhaltungen :
**EP-A- 0 021 345**
**EP-A- 0 042 980**
**EP-A- 0 049 111**
**DE-A- 2 201 063**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Jäger, Gerhard, Dr.**
**Gellerstrasse 18**
**D-5090 Leverkusen (DE)**
Erfinder : **Elbe, Hans-Ludwig, Dr.**
**Dasnoeckel 59**
**D-5600 Wuppertal 11 (DE)**
Erfinder : **Böckmann, Klaus, Dr.**
**Andreas-Gryphius-Strasse 7**
**D-5000 Köln 80 (DE)**
Erfinder : **Schulze, Andreas, Dr.**
**Voiswinkeler Strasse 35**
**D-5060 Bergisch-Gladbach (DE)**
Erfinder : **Büchel, Karl Heinz, Prof.Dr.**
**Dabringhausener Strasse 42**
**D-5093 Burscheid (DE)**
Erfinder : **Reinecke, Paul, Dr.**
**Lessingstrasse 11**
**D-5090 Leverkusen (DE)**
Erfinder : **Frohberger, Paul-Ernst, Dr.**
**Willi-Baumeister Strasse 5**
**D-5090 Leverkusen (DE)**
Erfinder : **Brandes, Wilhelm, Dr.**
**Eichendorffstrasse 3**
**D-5653 Leichlingen (DE)**
Erfinder : **Scheinpflug, Hans, Dr.**
**Am Thelenhof 15**
**D-5090 Leverkusen (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Die vorliegende Erfindung betrifft neue substituierte Azolylether-ketone und -carbinole, mehrere Verfahren zu ihrer Herstellung sowie ihre Verwendung als Fungizide.

Es ist bereits bekannt geworden, daß bestimmte substituierte 1-Azolyl-3,3-dimethyl-1-phenoxy-butan-2-one und -ole im allgemeinen gute fungizide Eigenschaften aufweisen (vergleiche DE-A 2 632 603, DE-A 2 632 602, DE-A 2 635 666 und DE-A 3 021 551 bzw. EP-A 0 042 980). Die Wirkung dieser Verbindungen ist jedoch, insbesondere bei niedrigen Aufwandmengen und -konzentrationen, in einigen Anwendungsbereichen nicht immer voll befriedigend. Dasselbe gilt auch für einige bisher noch nicht beschriebene 5-Aryloxy-5-azoly-3,3-dimethyl-1-penten-4-one und -ole DE-A 3 130 435).

Außerdem sind zahlreiche fungizid wirksame Azolyletherketone und -carbinole bekannt, in denen eine über CO oder CH(OH) mit dem verbleibenden Molekülteil verbundene Gruppe für Alkyl, Cycloalkyl, gegebenenfalls substituiertes Aryl oder gegebenenfalls substituiertes Aroxyalkyl steht (vgl. EP-A 0 021 345, DE-A 2 201 063 und EP-A 0 049 111). Es werden aber keine Verbindungen dieses Typs offenbart, in denen der betreffende Rest für substituiertes Cycloaklyl, verzweigtes Alkyl oder Halogenalkyl mit mehr als 4 Kohlenstoffatomen, über $—(CH_3)_2—$ gebundenes gegebenenfalls substituiertes Phenoxyethyl oder über $—C(CH_3)_2—CH_2—$ gebundenes gegebenenfalls substituiertes Phenyl steht.

Es wurden neue substituierte Azolylether-ketone und -carbinole der Formel

```
Ar-O-CH-B-R
     |
     Az
```
(I)

in welcher

Ar für gegebenenfalls einfach oder mehrfach, gleich oder verschieden substituiertes Phenyl steht, wobei als Substituenten genannt seien : Halogen, Alkyl mit 1 bis 4 Kohlenstoffatomen, Alkoxy und Alkylthio mit jeweils 1 bis 2 Kohlenstoffatomen ; Halogenalkyl, Halogenalkoxy und Halogenalkylthio mit jeweils 1 bis 2 Kohlenstoffatomen und 1 bis 5 gleichen oder verschiedenen Halogenatomen, Alkoxycarbonyl mit 1 bis 4 Kohlenstoffatomen im Alkylteil, Nitro, Cyano oder gegebenenfalls durch Halogen und/oder Alkyl mit 1 bis 2 Kohlenstoffatomen substituiertes Phenyl oder Phenoxy,

Az für 1,2,4-Triazol-1-yl, 1,2,4-Triazol-4-yl oder Imidazol-1-yl steht,

B für die Ketogruppe oder eine CH(OH)-Gruppierung steht und

R für einfach oder mehrfach, gleich oder verschieden substituiertes Cycloalkyl mit 3 bis 7 Kohlenstoffatomen steht, wobei als Substituenten genannt seien : Halogen, Alkyl mit 1 bis 4 Kohlenstoffatomen, Alkoxy mit 1 bis 2 Kohlenstoffatomen und Phenyl ; ferner für die Gruppierungen $—C(CH_3)_2—R^1$, $—C(CH_3)_2—CH_2CH_2—R^2$, $—C(CH_3)_2—(CH_2)_m—R^3$ und $—C(CH_3)_2—(CH_2)_n—R^4$ steht ; wobei

$R^1$ für geradkettiges oder verzweigtes Alkyl mit 2 bis 6 Kohlenstoffatomen, für Alkenyl mit 3 bis 6 Kohlenstoffatomen und Alkinyl mit 2 bis 4 Kohlenstoffatomen steht, ferner für die Aldehydgruppe und deren Derivate steht, wie Oxime, Oximether und Acetale, wie Dialkylacetale mit 1 bis 4 Kohlenstoffatomen in jedem Alkylteil oder für Dioxan und Dioxolan steht,

$R^2$ für gegebenenfalls einfach oder mehrfach, gleich oder verschieden substituiertes Phenoxy steht, wobei als Substituenten die bei Ar oben genannten Phenylsubstituenten infrage kommen ;

$R^3$ für gegebenenfalls einfach oder mehrfach, gleich oder verschieden substituiertes Phenyl steht, wobei als Substituenten die bei Ar oben genannten Phenylsubstituenten infrage kommen ;

$R^4$ für Fluor, Chlor oder Brom steht ;

m für ganze Zahlen von 1 bis 4 steht und

n für ganze Zahlen von 2 bis 4 steht,

sowie deren Säureadditionssalze und Metallsalzkomplexe gefunden.

Diejenigen Verbindungen der Formel (I), in denen B für die CH(OH)-Gruppe steht, besitzen zwei asymmetrische Kohlenstoffatome ; sie können deshalb in den beiden geometrischen Isomeren (erythro- und threo-Form) vorliegen, die in unterschiedlichen Mengenverhältnissen anfallen können.

In beiden Fällen liegen sie als optische Isomere vor. Verbindungen der Formel (I), in welcher R für substituiertes Cycloalkyl steht, können zusätzlich ein asymmetrisches Kohlenstoffatom besitzen, womit sich weitere geometrische Isomeren ergeben können. Sämtliche Isomeren werden erfindungsgemäß beansprucht.

Weiterhin wurde gefunden, daß man die neuen substituierten Azolylether-ketone und -carbinole der Formel (I) sowie deren Säureadditionssalze und Metallsalz-Komplexe erhält, wenn man

a) Halogenetherketone der Formel

```
Ar-O-CH-CO-R
     |
     Hal
```
(II)

in welcher

Ar und R die oben angegebene Bedeutung haben, und
Hal für Chlor oder Brom steht,
mit 1,2,4-Triazol oder Imidazol in Gegenwart eines Säurebindemittels und gegebenenfalls in Gegenwart eines Verdünnungsmittels umsetzt ; oder

b) Dihalogenketone der Formel

$$(Hal)_2—CH—CO—R \qquad (III)$$

in welcher Hal und R die oben angegebene Bedeutung haben, mit 1,2,4-Triazol oder Imidazol und mit einem Phenol der Formel

$$Ar—OH \qquad (IV)$$

in welcher Ar die oben angegebene Bedeutung hat, in Gegenwart eines Säurebindemittels und in Gegenwart eines Verdünnungsmittels umsetzt ; oder gegebenenfalls

c) die nach den Verfahren (a) oder (b) erhaltenen Azolylether-ketone der Formel

$$\underset{\displaystyle Az}{\text{Ar-O-}\overset{}{\text{CH}}\text{-CO-R}} \qquad (Ia)$$

in welcher Ar, Az und R die oben angegebene Bedeutung haben, nach bekannten Methoden in üblicher Weise reduziert, und gegebenenfalls zur Herstellung von Säureadditionssalzen und Metallsalzkomplexen an die so erhaltenen Verbindungen der Formel (I) anschließend eine Säure oder ein Metallsalz addiert.

Die neuen substituierten Azolylether-ketone und -carbinole der Formel (I) weisen starke fungizide Eigenschaften auf. Dabei zeigen überraschenderweise die erfindungsgemäßen Verbindungen eine bessere fungizide Wirksamkeit, als die aus dem Stand der Technik bekannten substituierten 1-Azolyl-3,3-dimethyl-1-phenoxy-butan-2-one und -ole, welche chemisch und wirkungsmäßig naheliegende Verbindungen sind. Die erfindungsgemäßen Stoffe stellen somit eine Bereicherung der Technik dar.

Die erfindungsgemäßen substituierten Azolylether-ketone und -carbinole sind durch die Formel (I) allgemein definiert.

Bevorzugt sind diejenigen Verbindungen der Formel (I), in denen

Ar für gegebenenfalls einfach bis dreifach, gleich oder verschieden substituiertes Phenyl steht, wobei als Substituenten genannt seien : Fluor, Chlor, Brom, Methyl, Ethyl, Isopropyl, Methoxy, Methlylthio, Trifluormethyl, Trifluormethoxy, Trifluormethylthio, Methoxycarbonyl, Ethoxycarbonyl, Nitro, Cyano, Phenyl, Chlorphenyl und Phenoxy ;

R für einfach bis dreifach, gleich oder verschieden substituiertes Cycloalkyl mit 3 bis 6 Kohlenstoffatomen steht, wobei als Substituenten genannt seien : Fluor, Chlor, Brom, Methyl, Ethyl, Methoxy, Ethoxy und Phenyl ; ferner für die Gruppierungen $—C(CH_3)_2—R^1$; $—C(CH_3)_2—CH_2CH_2—R^2$, $—C(CH_3)_2—(CH_2)_m—R^3$ und $—C(CH_3)_2—(CH_2)_n—R^4$ steht ; wobei

$R^1$ für Ethyl, n-Propyl, i-Propyl, n-Butyl, i-Butyl, sek. -Butyl, tert.-Butyl oder Neopentyl steht, weiterhin für Alkenyl mit 3 bis 6 Kohlenstoffatomen und Alkinyl mit 2 bis 4 Kohlenstoffatomen steht, ferner für die Aldehydgruppe und deren Derivate steht, wie Oxime und Oximether, zu letzteren seien genannt : Alkylether mit 1 bis 4 Kohlenstoffatomen, Alkenylether mit 2 bis 4 Kohlenstoffatomen, Alkinylether mit 2 bis 4 Kohlenstoffatomen und gegebenenfalls durch Halogen ein- bis dreifach substituierte Benzylether ; und Acetale, wie beispielsweise Dialkylacetale mit 1 bis 4 Kohlenstoffatomen in jedem Alkylteil, Dioxan und Dioxolan ;

$R^2$ für gegebenenfalls einfach bis dreifach, gleich oder verschieden substituiertes Phenoxy steht, wobei als Substituenten die bei Ar bereits genannten Phenylsubstituenten infrage kommen ;

$R^3$ für gegebenenfalls einfach bis dreifach, gleich oder verschieden substituiertes Phenyl steht, wobei als Substituenten die bei Ar bereits genannten Phenylsubstituenten infrage kommen ;

$R^4$ für Fluor oder Chlor steht ;

m für 1 oder 2 steht ;

n für 2 steht ;

Az und B für die in der Erfindungsdefinition angegebenen Bedeutungen stehen.

Verwendet man beispielsweise 1-Brom-1-(4-chlorphenoxy)-4-(2,4-dichlorphenyl)-3,3-dimethyl-2-butanon und 1,2,4-Triazol als Ausgangsstoffe, so kann der Reaktionsablauf durch das folgende Formelschema wiedergegeben werden (Verfahren a) :

(Siehe Formel Seite 4 f.)

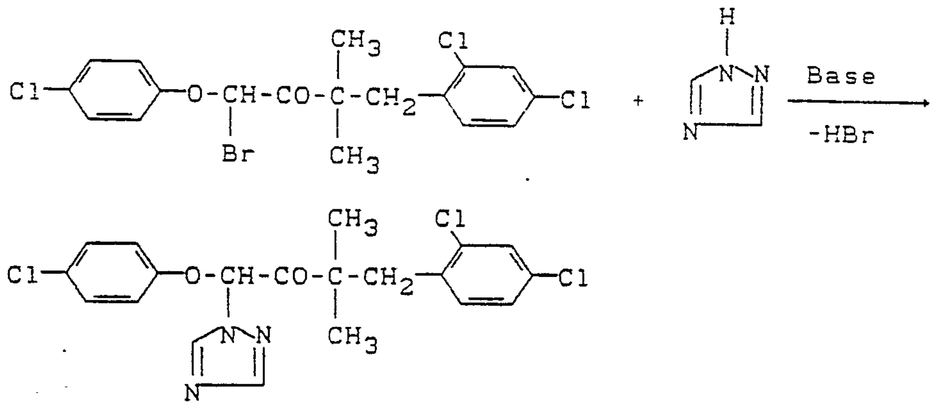


Verwendet man beispielsweise 1,1-Dichlor-3-(dioxan-2-yl)-3-methyl-2-butanon, 4-Chlorphenol und 1,2,4-Triazol als Ausgangsstoffe, so kann der Reaktionsablauf durch das folgende Formelschema wiedergegeben werden (Verfahren b) :

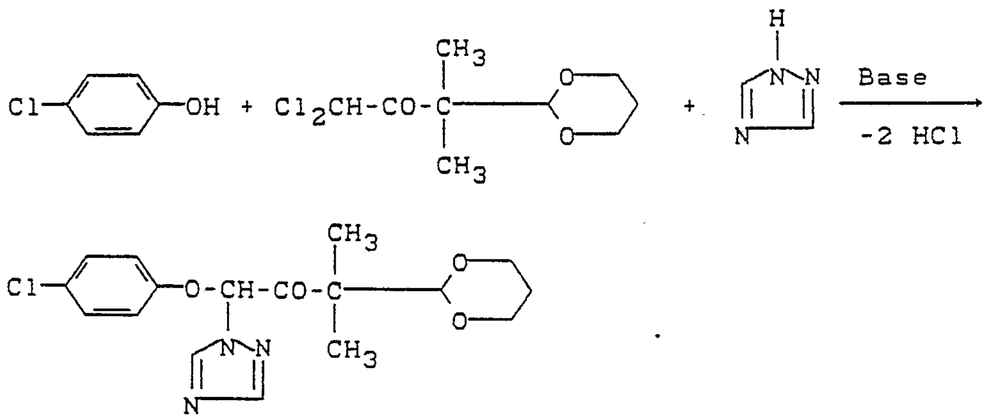


Verwendet man beispielsweise 1-(4-Chlorphenoxy)-4-(2,4-dichlorphenyl)-3,3-dimethyl-1-(1,2,4-triazol-1-yl)-2-butanon und Natriumborhydrid als Ausgangsstoffe, so kann der Reaktionsablauf durch das folgende Formelschema wiedergegeben werden (Verfahren c) :

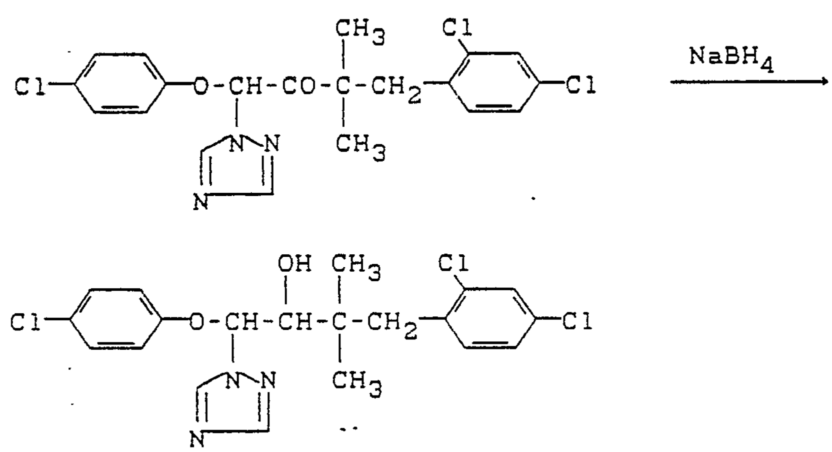


Die bei der Durchführung des erfindungsgemäßen Verfahrens (a) als Ausgangsstoffe zu verwendenden Halogenetherketone sind durch die Formel (II) allgemein definiert. In dieser Formel stehen A und R für die Reste, die bereits im Zusammenhang mit der Beschreibung der erfindungsgemäßen Stoffe der Formel (I) für diese Substituenten genannt wurden.

Die Halogenetherketone der Formel (II) sind noch nicht bekannt ; sie können jedoch in allgemein bekannter Art und Weise erhalten werden, indem man z. B. bei Etherketonen der Formel

$$Ar—O—CH_2—CO—R \qquad (V)$$

in welcher

Ar und R die oben angegebene Bedeutung haben, eines der beiden aktiven Wasserstoffatome in üblicher Weise gegen Chlor oder Brom austausch. Die entstehenden Halogenetherketone der Formel (II) können in allgemein bekannter Art und Weise enhalten werden, indem man Halogenketone der Formel

$$Hal—CH_2—CO—R \quad (VI)$$

Hal und R die oben angegebene Bedeutung haben,
mit Phenolen der Formel (IV) in Gegenwart einer starken Base, wie z. B. Kaliumcarbonat, und in Gegenwart eines inerten organischen Lösungsmittels, wie z. B. Aceton oder N,N-Dimethylformamid, bei Temperaturen zwischen 20 und 120 °C umsetzt.

Die Halogenketone der Formel (VI) sind teilweise bekannt (vergleiche z. B. DE-A 3 028 330) teilweise sind sie Gegenstand eigener älterer Patentanmeldungen, (vergleiche DE-A 3 145 857, DE-A 3 145 858, DE-A 3 209 431, DE-A 3 222 220 und DE-A 3 224 129) ; bzw. lassen sie sich nach im Prinzip bekannten Verfahren herstellen.

Die bei der Durchführung des erfindungsgemäßen Verfahrens (b) als Ausgangsstoffe zu verwendenden Dihalogenketone sind durch die Formel (III) allgemein definiert. In dieser Formel steht R für die Reste, die bereits im Zusammenhang mit der Beschreibung der erfindungsgemäßen Stoffe der Formel (I) für diesen Substituenten genannt wurden.

Die Dihalogenketone der Formel (III) sind noch nicht bekannt ; teilweise sind sie Gegenstand einer eigenen älteren Patentanmeldung (vergleiche DE-A 3 224 130) ; sie lassen sich nach im Prinzip bekannten Verfahren herstellen.

Die außerdem für die Durchführung des erfindungsgemäßen Verfahrens (b) als Ausgangsstoffe zu verwendenden Phenole sind durch die Formel (IV) allgemein definiert. In dieser Formel steht Ar für diejenigen Reste, die bereits im Zusammenhang mit der Beschreibung der erfindungsgemäßen Stoffe der Formel (I) für diesen Substituenten genannt wurden.

Die Phenole der Formel (IV) sind allgemein bekannte Verbindungen der organischen Chemie.

Die bei der Durchführung des Verfahrens (c) als Ausgangsstoffe zu verwendenden Azolyletherketone der Formel (Ia) sind erfindungsgemäße Stoffe.

Für die erfindungsgemäße Umsetzung gemäß Verfahren (a) kommen als Verdünnungsmittel inerte organische Lösungsmittel infrage. Hierzu gehören vorzugsweise Ketone, wie Diethylketon und insbesondere Aceton und Methylethylketon ; Nitrile, wie Propionitril, insbesondere Acetonitril ; Alkohole, wie Ethanol und Isopropanol ; Ether, wie Tetrahydrofuran oder Dioxan ; Benzol, Toluol, Formamide, wie insbesondere Dimethylformamid ; und halogenierte Kohlenwasserstoffe.

Die erfindungsgemäße Umsetzung gemäß Verfahren (a) wird in Gegenwart eines Säurebinders vorgenommen. Man kann alle üblicherweise verwendbaren anorganischen oder organischen Säurebinder zugeben, wie Alkalicarbonate, beispielsweise Natriumcarbonat, Kaliumcarbonat und Natriumhydrogencarbonat, oder wie niedere tertiäre Alkylamine, Cycloalkylamine oder Aralkylamine, beispielsweise Triethylamin, N,N-Dimethylcyclohexylamin, Dicyclohexylamin, N,N-Dimethylbenzylamin, weiterhin Pyridin und Diazabicyclooctan. Vorzugsweise verwendet man einen entsprechenden Ueberschuß an Triazol bzw. Imidazol.

Die Reaktionstemperaturen können beim Verfahren (a) in einem größeren Bereich variiert werden. Im allgemeinen arbeitet man zwischen etwa 20 bis etwa 150 °C, vorzugsweise bei 60 bis 120 °C. Bei Anwesenheit eines Lösungsmittels wird zweckmäßigerweise beim Siedepunkt des jeweiligen Lösungsmittels gearbeitet.

Bei der Durchführung des erfindungsgemäßen Verfahrens (a) setzt man auf 1 Mol der Verbindungen der Formel (II) vorzugsweise 1 bis 2 Mol Triazol bzw. Imidazol und 1 bis 2 Mol Säurebinder ein. Zu Isolierung der Verbindungen der Formel (I) wird das Lösungsmittel abdestilliert, der Rückstand mit einem organischen Solvens aufgenommen und mit Wasser gewaschen. Die organische Phase wird über Natriumsulfat getrocknet und im Vakuum vom Lösungsmittel befreit. Der Rückstand wich durch Destillation bzw. Umkristallisation oder Salzbildung und Umkristallisation gereinigt.

Für die erfindungsgemäße Umsetzung gemäß Verfahren (b) kommen als Verdünnungsmittel polare organische Lösungsmittel infrage. Hierzu gehören vorzugsweise chlorierte Kohlenwasserstoffe, wie Methylenchlorid oder Chloroform ; Alkohole, wie Ethanol, Propanol, n-Butanol oder tert.-Butanol ; Ketone wie Methylethylketon oder Aceton ; Ether, wie Tetrahydrofuran oder Dioxan ; und Nitrile, wie Acetonitril.

Die erfindungsgemäße Umsetzung gemäß Verfahren (b) wird in Gegenwart eines Säurebinders vorgenommen. Man kann alle üblicherweise verwendbaren anorganischen und organischen Säurebinder zugegeben. Hierzu gehören vorzugsweise die beim Verfahren (a) bereits genannten Solventien.

Die Reaktionstemperaturen können beim Verfahren (b) in einem größeren Bereich variiert werden. Im allgemeinen arbeitet man zwischen 0 un 150 °C, vorzugsweise zwischen 40 und 100 °C.

Bei der Durchführung des erfindungsgemäßen Verfahrens (b) setzt man auf 1 Mol Dihalogenketon der Formel (III) vorzugsweise 1 bis 1,2 Mol Phenol der Formel (IV) und 1 bis 1,2 Mol Triazol oder Imidazol sowie 2 bis 3 Mol Säurebinder ein. Die Isolierung der Endprodukte erfolgt in allgemein üblicher Art und Weise.

Die erfindungsgemäße Reduktion gemäß Verfahren (c) erfolgt in üblicher Weise, z. B. durch

Umsetzung mit komplexen Hydriden, gegebenenfalls in Gegenwart eines Verdünnungsmittels, oder durch Umsetzung mit Aluminiumisopropylat in Gegenwart eines Verdünnungsmittels.

Arbeitet man mit komplexen Hydriden, so kommen als Verdünnungsmittel für die erfindungsgemäße Umsetzung polare organische Lösungsmittel infrage. Hierzu gehören vorzugsweise Alkohole, wie Methanol, Ethanol, Butanol, Isopropanol und Ether, wie Diethylether oder Tetrahydrofuran. Die Reaktion wird im allgemeinen bei —10 bis + 30 °C, vorzugsweise bei 0 bis 20 °C durchgeführt. Hierzu setzt man auf 1 Mol des Ketons der Formel (Ia) etwa 1 Mol eines komplexen Hydrids, wie Natriumborhydrid oder Lithiumalanat, ein. Zur Isolierung der reduzierten Verbindungen der Formel (I) wird der Rückstand in verdünnter Salzsäure aufgenommen, anschließend alkalisch gestellt und mit einem organischen Lösungsmittel extrahiert. Die weitere Aufarbeitung erfolgt in üblicher Art und Weise.

Arbeitet man mit Aluminiumisopropylat, so kommen als Verdünnungsmittel für die erfindungsgemäße Umsetzung bevorzugt Alkohole, wie Isopropanol, oder inerte Kohlenwasserstoffe, wie Benzol, infrage. Die Reaktionstemperaturen können wiederum in einem größeren Bereich variiert werden ; im allgemeinen arbeitet man zwischen 20 und 120 °C, vorzugsweise bei 50 bis 100 °C. Zur Durchführung der Reaktion setzt man auf 1 Mol des Ketons der Formel (Ia) etwa 0,3 bis 2 Mol Aluminiumisopropylat ein. Zur Isolierung der reduzierten Verbindungen der Formel (I) wird das überschüssige Lösungsmittel im Vakuum entfernt und die entstandenen Aluminium-Verbindungen mit verdünnter Schwefelsäure oder Natronlauge zersetzt. Die weitere Aufarbeitung erfolgt in üblicher Weise.

Zur Herstellung von Säureadditionssalzen der Verbindungen der Formel (I) kommen alle physiologisch verträglichen Säuren infrage. Hierzu gehören vorzugsweise die Halogen wasserstoffsäuren, wie z. B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wei z. B. Essigsäure, Maleinsäure, Bernsteinsäure, Fumarsäure, Weinsäure, Zitronensäure, Salizylsäure, Sorbinsäure, Milchsäure, sowie Sulfonsäuren, wie z. B. p-Toluolsulfonsäure und 1,5-Naphthalindisulfonsäure.

Die Salze der Verbindungen der Formel (I) können in einfacher Weise nach üblichen Salzbildungsmethoden, z. B. durch Lösen einer Verbindung der Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel oder durch Umkristallisation gereinigt werden.

Zur Herstellung von Metallsalz-Komplexen der Verbindungen der Formel (I) kommen vorzugsweise Salze von Metallen der II. bis IV.-Haupt- und der I. und II. sowie IV. bis VIII. Nebengruppe infrage, wobei Kupfer, Zink, Mangan, Magnesium, Eisen und Nickel beispielshaft genannt sein. Als Anionen der Salze kommen solche in Betracht, die sich von physiologischen Säuren ableiten. Hierzu gehören vorzugsweise die Halogenwasserstoffsäuren, wie z. B. die Chlorwasserstoffsäure und die Bromwasserstoffsäure, ferner Phosphorsäure, Salpetersäure und Schwefelsäure.

Die Metallkomplexe der Verbindungen der Formel (I) können in einfacher Weise nach üblichen Verfahren erhalten werden, so z. B. durch Lösen des Metallsalzes in Alkohol, z. B. Ethanol, und Hinzufügen zur Verbindung der Formel (I). Man kann Metallkomplexe in bekannter Weise, z. B. durch Abfiltrieren, isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung solcher Pilze eingesetzt werden, die echte Mehltauerkrankungen hervorrufen, so zur Bekämpfungvon Erysiphe-Arten, wie z. B. gegen den Erreger des Gerstenmehltaus (Erysiphe graminis) und des Gurkenmehltaus (Spaerotheca fuliginea) ; ferner zur Bekämpfung des Braunrostes am Weisen (Puccinia recondita) und weiterer Getreidekrankheiten wie z. B. Cochliobolus sativus und Pyrenophora teres, zur Bekämpfung von Reiskrankheiten, wie Pyricularia oryzae und Pellicularia sasakii, sowie zur Bekämpfung des Apfelschorfes (Venturia inaequalis) und des Bohnenrostes (Uromyces appendiculatus). Daneben besitzen die erfindungsgemäßen Stoffe auch eine gute fungizide *in-vitro*-Wirkung.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä. sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also

Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen infrage : Aromaten, wie Xylol, Toluol, oder Alkyl-naphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone, wie Aceton, Methylethylketon, Methylisobutylketon oder Cyclohexanon, starke polare Lösungsmittel, wie Dimethylformamid und Dimethylsulfoxid, sowie Wasser ; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgase, wie Halogenkohlenwasserstoffe sowie Butan, Propan, Stickstoff und Kohlendioxid ; als feste Trägerstoffe kommen in Frage : z. B. natürliche Gesteinsmehle, wie Kaoline, Tonerden, Talkum, Kreide, Quarz, Attapulgit, Montmorillonit oder Diatomeeerde und synthetische Gesteinsmehle, wie hoch disperse Kieselsäure, Aluminiumoxid und Silikate ; als feste Trägerstoffe für Granulate kommen in Frage : z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material, wie Sägemehl, Kokosnußschalen, Maiskolben und Tabakstengeln ; als Emulgier- und/oder schaumerzeugende Mittel kommen in Frage : z. B. nichtionogene und anionische Emulgatoren, wie Polyoxyethylen-Fettsäure-ester, Polyoxyethylen-Fettalkohol-ether, z. B. Alkylarylpolyglykol-ether, Alkylsulfonate, Alkylsulfate, Arylsulfonate sowie Eiweißhydrolysate ; als Dispergiermittel kommen in Frage : z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo-, Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z. B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,000 1 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,000 01 bis 0,1 Gew.-%, vorzugsweise von 0,000 1 bis 0,02 Gew.-%, am Wirkungsort erforderlich.

## Herstellungsbeispiele

### Beispiel 1 und 2

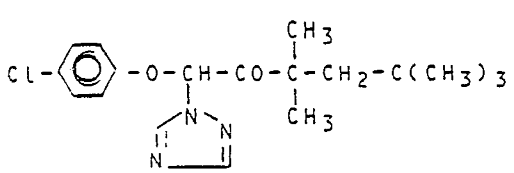

(Bsp.1)

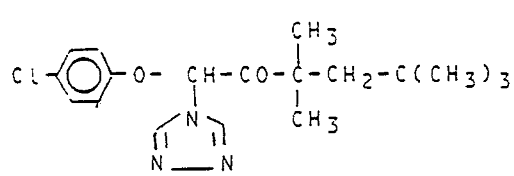

(Bsp.2)

(Verfahren a)

Eine Lösung von 49,7 g (0,72 Mol) 1,2,4-Triazol und 43,4 g (0,12 Mol) 1-Brom-1-(4-chlorphenoxy)-3,3,5,5-tetramethyl-hexan-2-on in 650 ml Acetonitril wird 5,5 Stunden zum Sieden erhitzt. Anschließend wird im Vakuum eingedampft, der Rückstand in 600 ml Essigester aufgenommen, dreimal mit je 100 ml Wasser gewaschen, über Natriumsulfat getrocknet und im Vakuum eingedampft. Das zurückbleibende viskose Oel (44 g) wird an Kieselgel mit Trichlormethan als Fließmittel chromatographiert. Man erhält 21,8 g (51,9 % der Theorie) 1-(4-Chlorphenoxy)-3,3,5,5-tetramethyl-1-(1,2,4-triazol-1-yl)-hexan-2-on als farblose Kristalle vom Schmelzpunkt 89-90 °C. Daneben werden 7,4 g (17,6 % der Theorie) des isomeren 1-(4-Chlorphenoxy)-3,3,5,5-tetramethyl-1-(1,2,4-triazol-4-yl)-hexan-2-on als farblose Kristalle vom Schmelzpunkt 146-48 °C erhalten.

Herstellung des Ausgangsproduktes

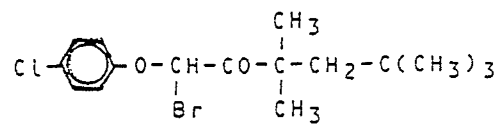

Zu einer Lösung von 67,9 g (0,24 Mol) 1-(4-Chlorphenoxy)-3,3,5,5-tetramethyl-hexan-2-on in 600 ml Dichlormethan werden bei 25 bis 30 °C 42,2 g (0,264 Mol) Brom unter kräftigem Rühren rasch zugetropft. Nach Beendigung der Bromzugabe läßt man noch 15 Minuten rühren, kühlt auf 10 °C ab und versetzt mit 500 ml eiskaltem Wasser. Die organische Phase wird abgetrennt, einmal mit 100 ml wäßriger Natriumbisulfit-Lösung und anschließend zweimal mit je 100 ml Wasser gewaschen. Nach dem Trocknen über Natriumsulfat wird das Lösungsmittel im Vakuum abdestilliert. Man erhält 86,8 g (100 % der Theorie) 1-Brom-1-(4-chlorphenoxy)-3,3,5,5-tetramethyl-hexan-2-on als schwach gelb gefärbte Kristalle vom Schmelzpunkt 72-73 °C.

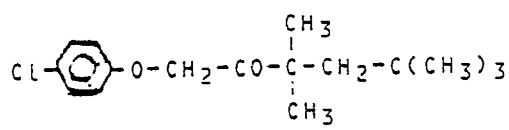

38,6 g (0,3 Mol) 4-Chlorphenol werden mit 41,4 g (0,3 Mol) Kaliumcarbonat und 57,2 g (0,3 Mol) 1-Chlor-3,3,5,5-tetramethyl-hexan-2-on in 350 ml N,N-Dimethylformamid 16 Stunden gerührt. Man kühlt anschließend auf 20 °C ab, filtriert vom ausgeschiedenen anorganischen Salz ab und dampft das Filtrat im Vakuum ein. Das zurückbleibende Oel (96,2 g) wird in 500 ml Essigester aufgenommen, dreimal mit je 100 ml 10 %-iger Sodalösung und zweimal mit je 100 ml Wasser gewaschen. Nach dem Trocknen über Natriumsulfat wird eingedampft und der ölige Rückstand (80 g) mit 50 ml Petrolether verrührt. Man erhält 68,6 g (81,1 % der Theorie) 1-(4-Chlorphenoxy)-3,3,5,5-tetramethyl-hexan-2-on als farblose Kristalle vom Schmelzpunkt 47-48 °C.

Beispiel 3

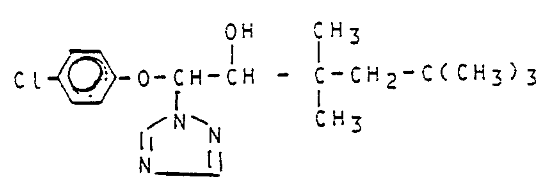

(Verfahren c)

Zu einer Lösung von 9,5 g (0,027 Mol) 1-(4-Chlorphenoxy)-3,3,5,5-tetramethyl-1-(1,2,4-triazol-1-yl)-hexan-2-on (Bsp. 1) in 95 ml Methanol werden bei 20 bis 30 °C unter Außenkühlung portionsweise 2 g (0,054 Mol) Natriumboranat eingetragen. Nach drei Stunden wird die Lösung durch Zugabe von Essigsäure auf einen pH-Wert von 6 gestellt. Man dampft anschließend im Vakuum ein, nimmt den Rückstand in 100 ml Essigester auf, wäscht dreimal mit je 20 ml Wasser, trocknet über Natriumsulfat und dampft im Vakuum ein. Man erhält 9,5 g (100 % der Theorie) 1-(4-Chlorphenoxy)-3,3,5,5-tetramethyl-1-(1,2,4-triazaol-1-yl)-hexan-2-ol als farbloses viskoses Oel mit einem Brechungsindes $n_D^{20}$ = 1,525 0.

Beispiel 4

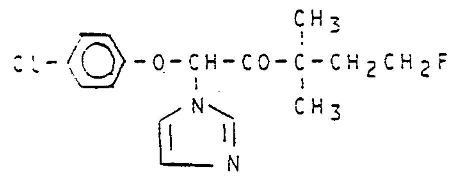

(Verfahren a)

Zu einem siedenden Gemisch aus 4,6 g (0,035 Mol) 4-Chlorphenol, 4,9 g (0,035 Mol) Kaliumcarbonat und 20 ml Aceton werden 5,9 g (0,035 Mol) 1-Chlor-3,3-dimethyl-5-fluorpentan-2-on zugetropft. Nach vierstündigem Erhitzen wird vom ausgeschiedenen Kaliumchlorid abfiltriert und das Filtrat unter vermindertem Druck eingedampft. Das als farbloses Oel verbleibende 1-(4-Chlorphenoxy)-3,3-dimethyl-5-fluor-pentan-2-on (9 g ; 100 % der Theorie) wird in 40 ml Dichlorethan gelöst und bei 20 °C innerhalb von 20 Minuten mit 5,9 g (0,037 Mol) Brom versetzt. Man läßt zwei Stunden bei 20 °C rühren, wäscht die Lösung dreimal mit jeweils 20 ml Wasser und destilliert das Lösungsmittel unter Vakuum ab. Als Rückstand verbleiben 11,8 g (100 % der Theorie) 1-Brom-1-(4-chlorphenoxy)-3,3-dimethyl-5-fluorpentan-2-on als schwach gelbliches Oel. Man nimmt in 20 ml Acetonitril auf und tropft die Lösung innerhalb von 20 Minuten zu einer siedenden Lösung von 14,3 g (0,21 Mol) Imidazol in 120 ml Acetonitril. Nach vierstündigem Erhitzen unter Rückfluß wird das Lösungsmittel im Vakuum abdestilliert, der Rückstand in 200 ml Essigester aufgenommen, dreimal mit jeweils 50 ml Wasser gewaschen, über Natriumsulfat getrocknet und unter vermindertem Druck eingedampft. Nach dem Filtrieren des verbleibenden Oels (10,8 g) über eine Kieselgelsäule (Essigester : Trichlorethan, 1 : 1) erhält man durch Eindampfen des Eluats 8,0 g (70,2 % der Theorie) 1-(4-Chlorphenoxy)-3,3-dimethyl-5-fluor-1-(imidazol-1-yl)-pentan-2-on als farblose Kristalle vom Schmelzpunkt 77-78 °C.

Beispiel 5

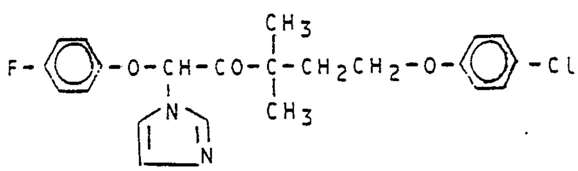

(Verfahren a)

Eine Lösung von 40,2 g (0,591 Mol) Imidazol und 43,0 g (0,1 Mol) 1-Brom-5-(4-chlorphenoxy)-3,3-dimethyl-1-(4-fluorphenoxy)-pentan-2-on in 600 ml Acetonitril wird 4 Stunden zum Sieden erhitzt. Man dampft die Lösung anschließend im Vakuum ein, nimmt den Rückstand in 400 ml Essigester auf und wäscht mit je 100 ml Wasser. Nach dem Trocknen der organischen Phase über Natriumsulfat wird unter vermindertem Druck eingedampft und der verbleibende feste Rückstand mit Diethylether verrührt. Man erhält 34,3 g (83,5 % der Theorie) 5-(4-Chlorphenoxy)-3,3-dimethyl-1-(4-fluorphenoxy)-1-(imidazol-1-yl)-pentan-2-on als farblose Kristalle vom Schmelzpunkt 120-121 °C.

Herstellung des Ausgangsproduktes

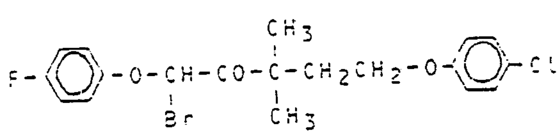

34,3 g (0,214 Mol) Brom werden rasch zu einer Lösung von 69,1 g (0,197 Mol) 5-(4-Chlorphenoxy)-3,3-dimethyl-1-(4-fluorphenoxy)-pentan-2-on in 600 ml Dichlormethan zugetropft. Nach Beendigung der Bromzugabe wird noch 15 Minuten nachgerührt und die Lösung anschließend mit 400 ml Eiswasser versetzt. Die organische Phase wird abgetrennt, einmal mit 100 ml wäßriger Natriumbisulfit-Lösung und zweimal mit je 100 ml Wasser gewaschen. Die Lösung wird über Natriumsulfat getrocknet und anschließend im Vakuum eingedampft. Man erhält 85,9 g (100 % der Theorie) 1-Brom-5-(4-chlorphenoxy)-3,3-dimethyl-1-(4-fluorphenoxy)-pentan-2-on als farblose Kristalle vom Schmelzpunkt 92-93 °C.

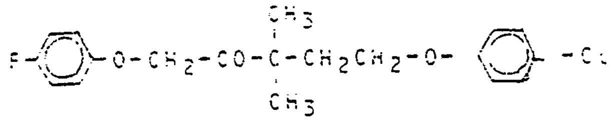

68,8 g (0,25 Mol) 1-Chlor-5-(4-chlorphenoxy)-3,3-dimethylpentan-2-on werden mit 28 g (0,25 Mol) 4-Fluorphenol und 34,5 g (0,25 Mol) gepulvertem Kaliumcarbonat in 600 ml Aceton 13 Stunden zum Sieden erhitzt. Anschließend wird von anorganischen Salzen abfiltriert, das Filtrat im Vakuum eingedampft und der Rückstand in Diethylether aufgenommen. Nach Zugabe von Petrolether erhält man 69,3 g (79 % der Theorie) 5-(4-Chlorphenoxy)-3,3-dimethyl-1-(4-fluorphenoxy)-pentan-2-on als farblose Kristalle vom Schmelzpunkt 71-74 °C.

Beispiel 6

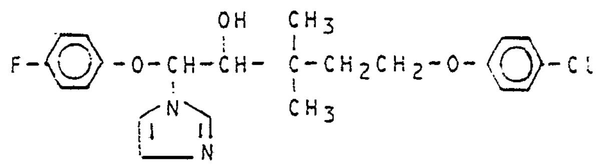

(Verfahren c)

Zu einer Lösung von 14,2 g (0,034 Mol) 5-(4-Chlorphenoxy)-3,3-dimethyl-1-(4-fluorphenoxy)-1-(imidazol-1-yl)-pentan-2-on (Bsp. 5) in 140 ml Methanol werden bei 20 bis 30 °C unter Außenkühlung portionsweise 2,6 g (0,068 Mol) Natriumboranat eingetragen. Nach zwei Stunden wird die Lösung durch Zugabe von Essigsäure auf einen pH-Wert von 6 gebracht und im Vakuum eingedampft. Der Rückstand wird in 200 ml Essigester aufgenommen, zweimal mit je 50 ml Wasser gewaschen, über Natriumsulfat getrocknet und eingedampft. Durch Verreiben des festen Rückstandes mit Petrolether erhält man 12,3 g (86,6 % der Theorie) 5-(4-Chlorphenoxy)-3,3-dimethyl-1-(4-fluorphenoxy)-1-(imidazol-1-yl)-pentan-2-ol als farblose Kristalle vom Schmelzpunkt 88-93 °C.

Beispiel 7

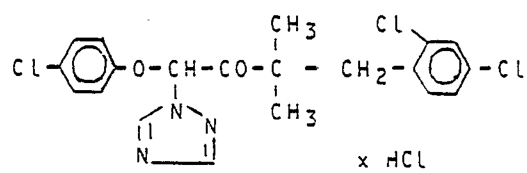

x HCl

(Verfahren a)

11 g (0,16 Mol) 1,2,4-Triazol und 22 g (0,16 Mol) Kaliumcarbonat werden in 200 ml Toluol 1 Stunde bei 90 °C gerührt. Dazu werden 35 g (0,078 Mol) 1-Brom-1-(4-chlorphenoxy)-4-(2,4-dichlorphenyl)-3,3-dimethyl-butan-2-on in 100 ml Toluol getropft. Man läßt das Reaktionsgemisch 10 Stunden bei 90 °C nachrühren, kühlt anschließend auf Raumtemperatur ab und versetzt mit 200 ml Wasser. Die organische Phase wird abgetrennt, über Natriumsulfat getrocknet und eingeengt. Der Rückstand wird säulenchromatographisch gereinigt. Man erhält 7,8 g 1-(4-Chlorphenoxy)-4-(2,4-dichlorphenyl)-3,3-dimethyl-1-(1,2,4-triazol-1-yl)-butan-2-on als hochviskoses Oel.

3,9 g dieses hochviskosen Oels werden mit 15 ml gesättigter etherischer Salzsäure versetzt. Das ausgefallene Salz wird abgesaugt, mit wenig Ether gewaschen und bei 40 °C im Vakuum getrocknet. Man erhält 3,3 g (78,2 % der Theorie) 1-(4-Chlorphenoxy)-4-(2,4-dichlorphenyl)-3,3-dimethyl-1-(1,2,4-triazol-1-yl)-butan-2-on-hydrochlorid vom Schmelzpunkt 130-135 °C.

Herstellung des Ausgangsproduktes

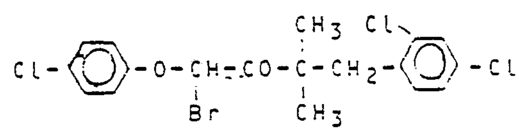

57 g (0,15 Mol) 1-(4-Chlorphenoxy)-4-(2,4-dichlorphenyl)-3,3-dimethyl-butan-2-on, gelöst in 400 ml Chloroform, werden bei Raumtemperatur mit 24,5 g (0,15 Mol) Brom versetzt. Man läßt das Reaktionsgemisch nach beendeter Zugabe 30 Minuten nachrühren und engt danach ein. Man erhält 67,6 g (100 % der Theorie) rohes 1-Brom-1-(4-chlorphenoxy)-4-(2,4-dichlorphenyl)-3,3-dimethyl-butan-2-on, das direkt weiter umgesetzt wird.

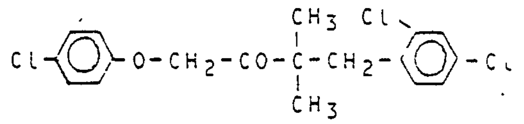

28,2 g (0,22 Mol) 4-Chlorphenol und 30,4 g (0,22 Mol) Kaliumcarbonat werden in 120 ml Toluol 2 Stunden unter Rückfluß am Wasserabscheider erhitzt. Bei 60 °C läßt man eine Lösung von 57 g (0,18 Mol) 1-Brom-4-(2,4-dichlorphenyl)-3,3-dimethyl-butan-2-on in 200 ml Toluol zutropft. Das Reaktionsgemisch wird dann 5 Stunden bei 100 °C nachgerührt. Man läßt abkühlen, saugt vom anorganischen Rückstand ab, wäscht die Toluolphase mit verdünnter Natronlauge und anschließend mit Wasser, trocknet über Natriumsulfat und engt ein. Man erhält 57,4 g (70,2 % der Theorie) 1-(4-Chlorphenoxy)-4-(2,4-dichlorphenyl)-3,3-dimethyl-butan-2-on vom Brechungsindex $n_D^{20}$ = 1,575 3.

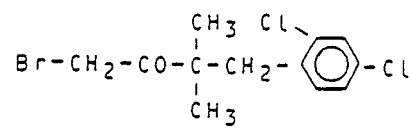

Zu 64,5 g (0,26 Mol) 4-(2,4-Dichlorphenyl)-3,3-dimethylbutan-2-on in 600 ml Chloroform läßt man bei Raumtemperatur 13,4 ml (0,26 Mol) Brom tropfen. Das Reaktionsgemisch wird 1 Stunde bei Raumtemperatur nachgerührt und eingeengt. Man erhält 84 g (100 % der Theorie) rohes 1-Brom-4-(2,4-dichlorphenyl)-3,3-dimethyl-butan-2-on, das direkt weiter umgesetzt wird.

Beispiel 8

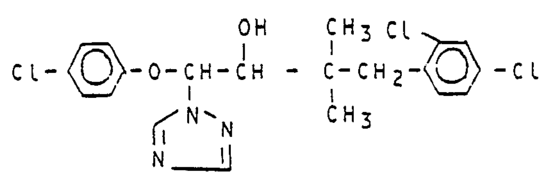

(Verfahren c)

3,5 g (0,008 Mol) 1-(4-Chlorphenoxy)-4-(2,4-dichlorphenyl)-3,3-dimethyl-1-(1,2,4-triazol-1-yl)-butan-2-on (vgl. Bsp. 7) werden bei — 10 °C in 50 ml Methanol gelöst und mit 0,1 g (0,002 6 Mol) Natriumborhydrid in 2 ml Wasser versetzt. Man läßt das Reaktionsgemisch 1 Stunde ohne Kühlung nachrühren und engt danach im Vakuum ein. Der Rückstand wird mit Methylenchlorid/Wasser versetzt. Die organische Phase wird abgetrennt, über Natriumsulfat getrocknet und eingeengt. Man erhält 3,2 g (91,4 % der Theorie) 1-(4-Chlorphenoxy)-4-(2,4-dichlorphenyl)-3,3-dimethyl-1-(1,2,4-triazol-1-yl)-butan-2-ol vom Schmelzpunkt 60-65 °C.

Beispiel 9

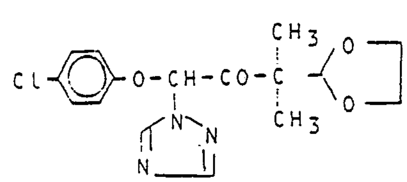

(Verfahren b)

Zu 43 g (0,19 Mol) 1,1-Dichlor-3-(dioxolan-2-yl)-3-methyl-butan-2-on und 78,4 g (0,57 Mol) Kaliumcarbonat in 300 ml Aceton gibt man unter Rückfluß ein Gemisch aus 14,4 g (0,21 Mol) 1,2,4-Triazol und 26,9 g (0,21 Mol) 4-Chlorphenol in 100 ml Aceton. Man läßt das Reaktionsgemisch 10 Std. unter Rückfluß nachrühren, kühlt anschließend ab und saugt vom anorganischen Rückstand ab. Das Filtrat

wird eingeengt, der Rückstand in Methylenchlorid aufgenommen, mit verdünnter Natronlauge und mit Wasser gewaschen, über Natriumsulfat getrocknet und im Vakuum eingeengt. Der Rückstand wird säulenchromatographisch gereinigt (Kieselgel/Essigester : Cyclohexan = 3 : 1). Man erhält 19 g (28,4 % der Theorie) 1-(4-Chlorphenoxy)-3-(dioxolan-2-yl)-3-methyl-1-(1,2,4-triazol-1-yl)-butan-2-on vom Brechungsindex $n_D^{20} = 1,499\ 4$.

Herstellung des Ausgangsproduktes

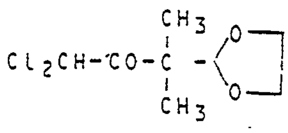

36,8 g (0,2 Mol) 4,4-Dichlor-2,2-dimethyl-3-ketobutanal werden mit 13,6 g (0,22 Mol) Ethylenglykol und 2,2 g p-Toluolsulfonsäure in einem Gemisch aus 180 ml Toluol und 160 ml n-Butanol 1,5 Stunden am Wasserabscheider unter Rückfluß erhitzt. Man läßt abkühlten und engt im Vakuum ein. Der Rückstand wird in Methylenchlorid aufgenommen, mit Wasser gewaschen, über Natriumsulfat getrocknet und eingeengt. Man erhält 43,6 g (96 % der Theorie) 1,1-Dichlor-3-(di-oxolan-2-yl)-3-methyl-butan-2-on vom Brechungsindex $n_D^{20} = 1,463\ 1$.

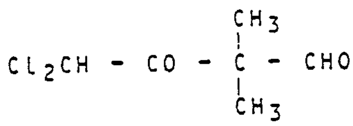

141 g (1 Mol) 1-(N-Morpholino)-isobuten werden in 470 ml Diethylether gelöst. Unter Eiskühlung tropft man 147,5 g (1 Mol) Dichloracetylchlorid langsam zu. Das Reaktionsgemisch wird anschließend 24 Stunden bei Raumtemperatur gerührt und danach mit 150 ml Wasser sowie Natriumhydrogencarbonatlösung versetzt, bis ein pH-Wert von 5 bis 6 erreicht ist. Man läßt eine weitere Stunde rühren und trennt danach die Etherphase ab. Die wässrige Phase wird mit Ether und anschließend mit Methylenchlorid extrahiert. Die vereinigten organischen Phasen werden über Natriumsulfat getrocknet, eingeengt und der Rückstand im Vakuum destilliert. Man erhält 155,6 g (85 % der Theorie) 4,4-Dichlor-2,2-dimethyl-3-ketobutanal vom Siedepunkt 118 °C/0,2 mbar.

Beispiel 10

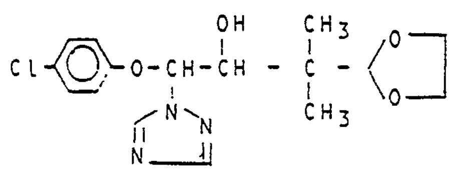

(Verfahren c)

7 g (0,02 Mol) 1-(4-Chlorphenoxy)-3-(dioxolan-2-yl)-3-methyl-1-(1,2,4-triazol-1-yl)-butan-2-on (Bsp. 9) werden in 70 ml Methanol gelöst und mit einer Lösung von 0,23 g (0,006 Mol) Natriumborhydrid in 5 ml Wasser versetzt. Man läßt das Reaktionsgemisch 2 Stunden nachrühren, stellt mit verdünnter Salzsäure einen pH-Wert von 6 bis 7 ein und engt im Vakuum ein. Der Rückstand wird in Methylenchlorid aufgenommen, mit Wasser gewaschen, über Natriumsulfat getrocknet und eingeengt. Man erhält 5,8 g (82,9 % der Theorie) 1-(4-Chlorphenoxy)-3-(dioxolan-2-yl)-3-methyl-1-(1,2,4-triazol-1-yl)-butan-2-ol vom Brechungsindex $n_D^{20}$ 1,538 9.

In entsprechender Weise und analog den angegebenen Verfahren werden die Verbindungen der allgemeinen Formel

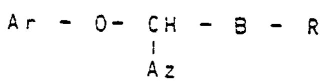

erhalten :

| Bsp. Nr. | Ar | Az | B | R | Fp(°C)bzw. $n_D^{20}$ |
|---|---|---|---|---|---|
| 11 | 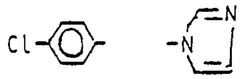 | | CO | $-C(CH_3)_2-CH_2-$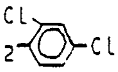 | 213(xHCl) |
| 12 | 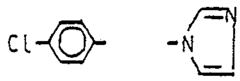 | | CO | $-C(CH_3)_2-CH_2-$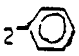 | zähes Oel |
| 13 | 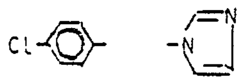 | | CO | $-C(CH_3)_2-CH_2-$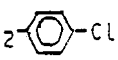 | 174-75(xHCl) |
| 14 | 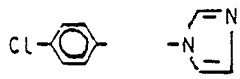 | | CO | 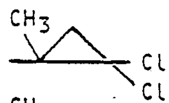 | 40 |
| 15 | 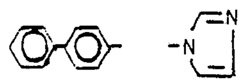 | | CO | 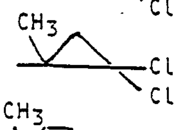 | 164 |
| 16 | 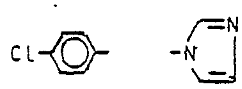 | | CO | 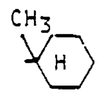 | zähes Oel |
| 17 | 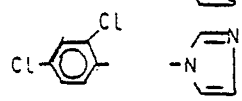 | | CO | 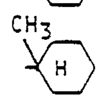 | zähes Oel |
| 18 | 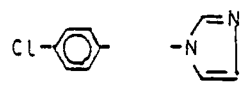 | | CO | 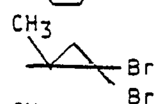 | 47 |
| 19 | 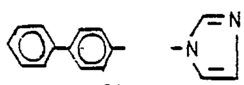 | | CO | 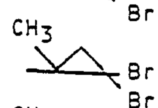 | 178 |
| 20 | 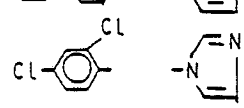 | | CO | 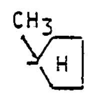 | zähes Oel |
| 21 | 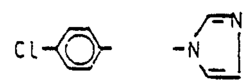 | | CO | 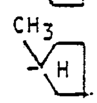 | zähes Oel |
| 22 | 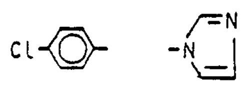 | | CO | $-C(CH_3)_2-C_3H_7-n$ | 1,5345 |
| 23 | 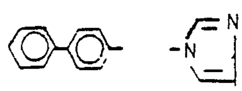 | | CO | $-C(CH_3)_2-C_3H_7-n$ | 65 |
| 24 | 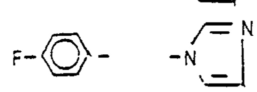 | | CO | $-C(CH_3)_2-C_3H_7-i$ | 1,5195 |
| 25 | 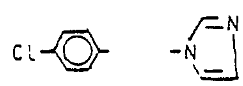 | | CO | $-C(CH_3)_2-CH_2CH_2Cl$ | Harz |
| 26 | 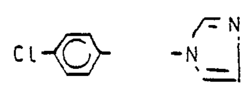 | | CO | $-C(CH_3)_2-C_4H_9-n$ | 1,5324 |
| 27 | 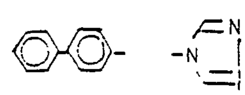 | | CO | $-C(CH_3)_2-C_3H_7-i$ | 127 |

(Fortsetzung)

| Bsp. Nr. | Ar | Az | B | R | Fp(°C) bzw. $n_D^{20}$ |
|---|---|---|---|---|---|
| 28 | 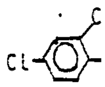 | 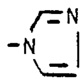 | CO | $-C(CH_3)_2-C_3H_7-i$ | Harz |
| 29 | 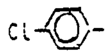 | 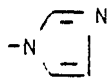 | CO | $-C(CH_3)_2-C_3H_7-i$ | Harz |
| 30 | 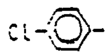 | 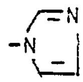 | CO | $-C(CH_3)_2-CH_2-C(CH_3)_3$ | 83-85 |
| 31 | 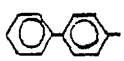 | 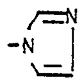 | CO | $-C(CH_3)_2-CH_2-C(CH_3)_3$ | 128 |
| 32 | 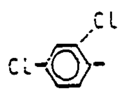 | 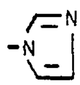 | CO | $-C(CH_3)_2-CH_2-C(CH_3)_3$ | 84 |
| 33 | 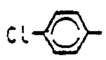 | 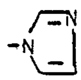 | CO | $-C(CH_3)_2-CH_2CH_2O-$ 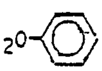 | Harz |
| 34 | 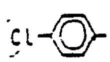 | 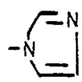 | CO | $-C(CH_3)_2-CH_2CH_2O$ 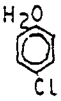 | Harz |
| 35 | 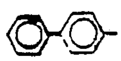 | 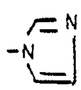 | CO | $-C(CH_3)_2-CH_2CH_2-O$ 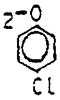 | 102-03 |
| 36 | 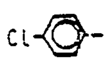 | 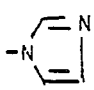 | CO | $-C(CH_3)_2-CH_2CH_2-O$ 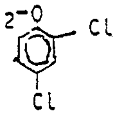 | Harz |
| 37 | 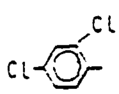 | 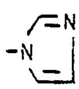 | CO | $-C(CH_3)_2-CH_2CH_2-O$ 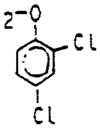 | 1,5822 |
| 38 | 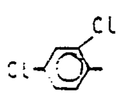 | 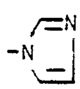 | CO | $-C(CH_3)_2-CH_2CH_2-O$ 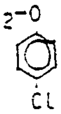 | 1,5746 |
| 39 | 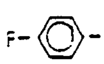 | 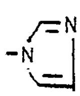 | CO | $-C(CH_3)_2-CH_2CH_2-O$ 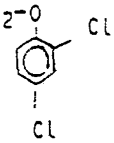 | 1,5629 |
| 40 | 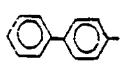 | 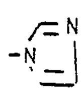 | CO | $-C(CH_3)_2-CH_2CH_2-O$ 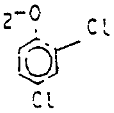 | 1,5950 |

(Fortsetzung)

| Bsp. Nr. | Ar | Az | B | R | Fp(°C) bzw. $n_D^{20}$ |
|---|---|---|---|---|---|
| 41 | 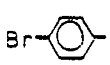 | | CO | 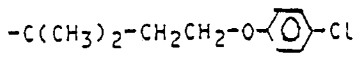 | 108-09 |
| 42 | 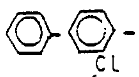 | | CO | 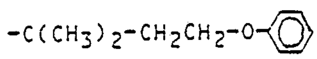 | 95-96 |
| 43 | 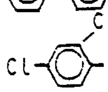 | | CO | 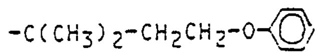 | Harz |
| 44 | 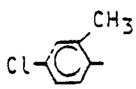 | | CO | 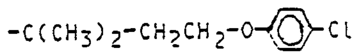 | Harz |
| 45 | 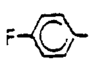 | | CO | 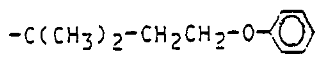 | 88-90 |
| 46 | 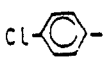 | | CO | 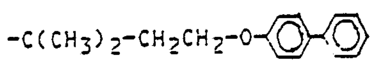 | Harz |
| 47 | 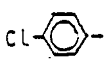 | | CO | 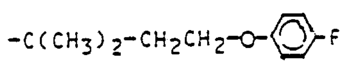 | zähes Oel |
| 48 | 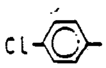 | | CO | 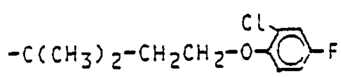 | zähes Oel |
| 49 | 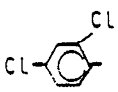 | | CO | 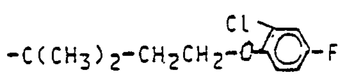 | zähes Oel |
| 50 | 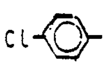 | | CH(OH) | 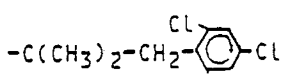 | 60-65 |
| 51 | 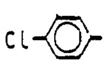 | | CH(OH) | 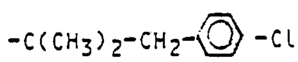 | 156-59 |
| 52 | 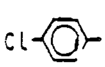 | | CH(OH) | 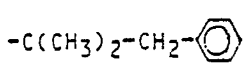 | 144 |
| 53 | 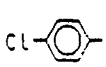 | | CH(OH) | 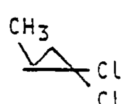 | 123 |
| 54 | 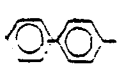 | | CH(OH) | 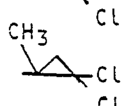 | 214 |
| 55 | 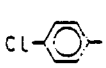 | | CH(OH) | 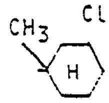 | 145 |
| 56 | 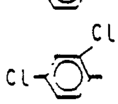 | | CH(OH) | 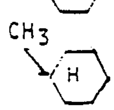 | zähes Oel |

(Fortsetzung)

| Bsp. Nr. | Ar | Az | B | R | Fp(°C) bzw. $n_D^{20}$ |
|---|---|---|---|---|---|
| 57 | 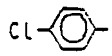 | 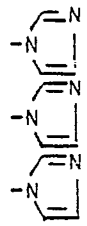 | CH(OH) | $-C(CH_3)_2-C_3H_7-n$ | 86-90 |
| 58 | 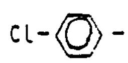 | | CH(OH) | $-C(CH_3)_2-CH_2CH_2Cl$ | Harz |
| 59 | 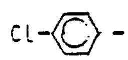 | | CH(OH) | $-C(CH_3)_2-C_4H_9-n$ | 1,5273 |
| 60 | 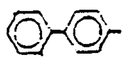 | 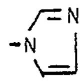 | CH(OH) | $-C(CH_3)_2-C_3H_7-i$ | 138-40 |
| 61 | 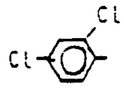 | 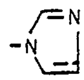 | CH(OH) | $-C(CH_3)_2-C_3H_7-i$ | zähes Oel |
| 62 | 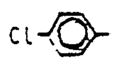 | 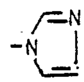 | CH(OH) | $-C(CH_3)_2-CH_2CH_2Cl$ | 102-03 |
| 63 | 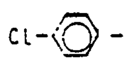 | 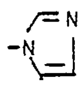 | CH(OH) | $-C(CH_3)_2-CH_2-C(CH_3)_3$ | 137-38 |
| 64 | 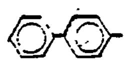 | 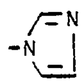 | CH(OH) | $-C(CH_3)_2-CH_2-C(CH_3)_3$ | 160 |
| 65 | 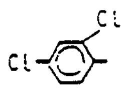 | 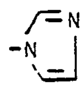 | CH(OH) | $-C(CH_3)_2-CH_2-C(CH_3)_3$ | Harz |
| 66 | 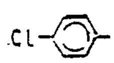 | 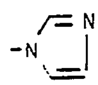 | CH(OH) | $-C(CH_3)_2-CH_2CH_2-O-$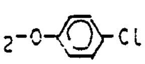 | 114-16 |
| 67 | 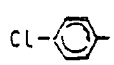 | 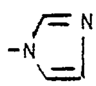 | CH(OH) | $-C(CH_3)_2-CH_2CH_2-O-$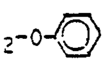 | 118-24 |
| 68 | 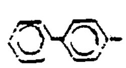 | 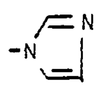 | CH(OH) | $-C(CH_3)_2-CH_2CH_2-O-$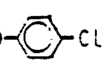 | 138-40 |
| 69 | 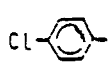 | 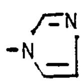 | CH(OH) | $-C(CH_3)_2-CH_2CH_2O-$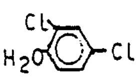 | 147-51 |
| 70 | 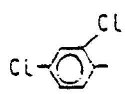 | 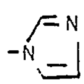 | CH(OH) | $-C(CH_3)_2-CH_2CH_2-O-$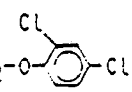 | Harz |
| 71 | 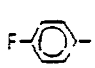 | 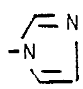 | CH(OH) | $-C(CH_3)_2-CH_2CH_2-O-$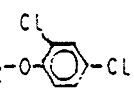 | 111-13 |
| 72 | 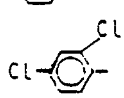 | 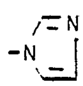 | CH(OH) | $-C(CH_3)_2-CH_2CH_3-O-$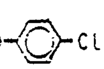 | Harz |
| 73 | 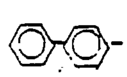 | 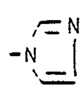 | CH(OH) | $-C(CH_3)_2-CH_2CH_2-O-$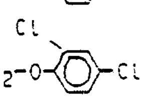 | 159-60 |

(Fortsetzung)

| Bsp. Nr. | Ar | Az | B | R | Fp(°C) bzw. $n_D^{20}$ |
|---|---|---|---|---|---|
| 74 | 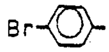 | 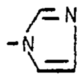 | CH(OH) | $-C(CH_3)_2-CH_2CH_2-O-$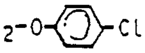 | 138-43 |
| 75 | 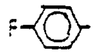 | 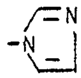 | CH(OH) | $-C(CH_3)_2-CH_2CH_2-O-$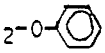 | Harz |
| 76 | 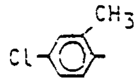 | 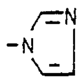 | CH(OH) | $-C(CH_3)_2-CH_2CH_2-O-$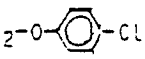 | 64-66 |
| 77 | 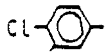 | 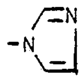 | CH(OH) | $-C(CH_3)_2-CH_2CH_2-O-$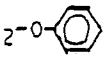 | Harz |
| 78 | 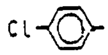 | 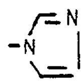 | CH(OH) | $-C(CH_3)_2-CH_2CH_2-O-$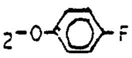 | Harz |
| 79 | 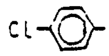 | 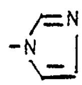 | CH(OH) | $-C(CH_3)_2-CH_2CH_2-O-$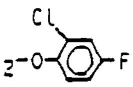 | Harz |
| 80 | 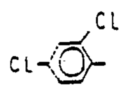 | 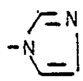 | CH(OH) | $-C(CH_3)_2-CH_2CH_2-O-$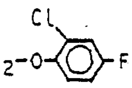 | Harz |
| 81 | 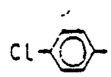 | 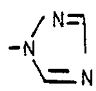 | CO | $-C(CH_3)_2-CH_2-$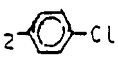 | 139-41(xHCl) |
| 82 | 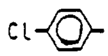 | 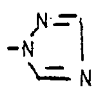 | CO | $-C(CH_3)_2-CH_2-$ | zähes Oel |
| 83 | 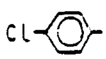 | 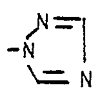 | CO | $-C(CH_3)_2-C_3H_7-n$ | 1,5312 |
| 84 | 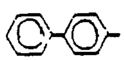 | 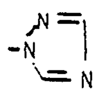 | CO | $-C(CH_3)_2-C_3H_7-n$ | 75 |
| 85 | 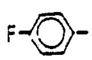 | 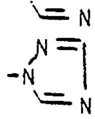 | CO | $-C(CH_3)_2-C_3H_7-i$ | 1,5116 |
| 86 | 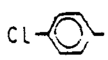 | 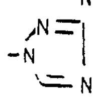 | CO | $-C(CH_3)_2-C_4H_9-n$ | 1,5250 |
| 87 | 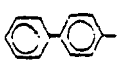 | 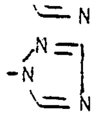 | CO | $-C(CH_3)_2-C_3H_7-i$ | 121-22 |
| 88 | 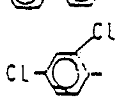 | 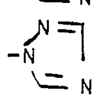 | CO | $-C(CH_3)_2-C_3H_7-i$ | 59 |
| 89 | 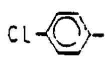 | 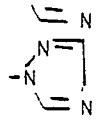 | CO | $-C(CH_3)_2-C_3H_7-i$ | 67 |

(Fortsetzung)

| Bsp. Nr. | Ar | Az | B | R | Fp(°C) bzw. $n_D^{20}$ |
|---|---|---|---|---|---|
| 90 | 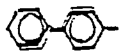 | 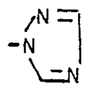 | CO | $-C(CH_3)_2-CH_2-C(CH_3)_3$ | 112 |
| 91 | 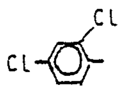 | 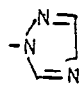 | CO | $-C(CH_3)_2-CH_2-C(CH_3)_3$ | 78 |
| 92 | 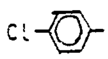 | 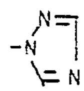 | CO | $-C(CH_3)_2-CHO$ | 1,5350 |
| 93 | 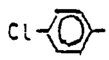 | 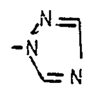 | CO | $-C(CH_3)_2-CH=NOCH_3$ | 1,5303 |
| 94 | 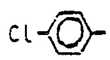 | 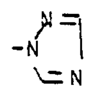 | CO | $-C(CH_3)_2-$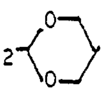 | zähes Oel |
| 95 | 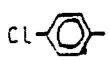 | 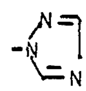 | CO | $-C(CH_3)_2-CH_2CH_2-O-$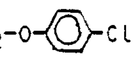 | 87-89 |
| 96 | 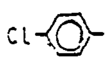 | 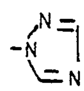 | CO | $-C(CH_3)_2-CH_2CH_2-O-$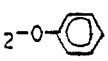 | Harz |
| 97 | 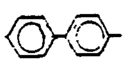 | 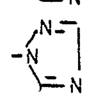 | CO | $-C(CH_3)_2-CH_2CH_2-O-$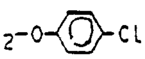 | 103-04 |
| 98 | 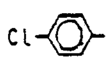 | 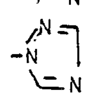 | CO | $-C(CH_3)_2-CH_2CH_2-O-$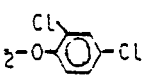 | Harz |
| 99 | 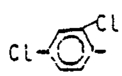 | 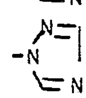 | CO | $-C(CH_3)_2-CH_2CH_2-O-$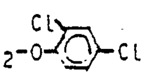 | 109-10 |
| 100 | 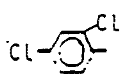 | 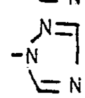 | CO | $-C(CH_3)_2-CH_2CH_2-O-$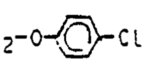 | 1,5720 |
| 101 | 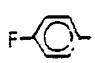 | 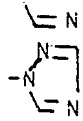 | CO | $-C(CH_3)_2-CH_2CH_2-O-$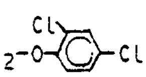 | 1,5608 |
| 102 | 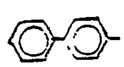 | 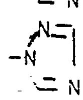 | CO | $-C(CH_3)_2-CH_2CH_2-O-$ | 88 |
| 103 | 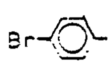 | 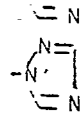 | CO | $-C(CH_3)_2-CH_2CH_2-O-$ | 94-95 |
| 104 | 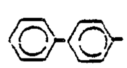 | 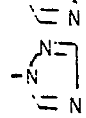 | CO | $-C(CH_3)_2-CH_2CH_2-O-$ | 86-88 |
| 105 | 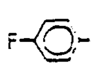 | 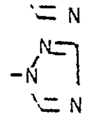 | CO | $-C(CH_3)_2-CH_2CH_2-O-$ | 107-08 |

(Fortsetzung)

| Bsp. Nr. | Ar | Az | B | R | Fp(°C) bzw. $n_D^{20}$ |
|---|---|---|---|---|---|
| 106 | 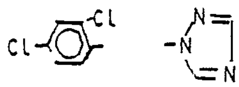 | | CO | $-C(CH_3)_2-CH_2CH_2-O-$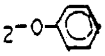 | Harz |
| 107 | 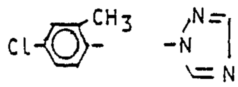 | | CO | $-C(CH_3)_2-CH_2CH_2-O-$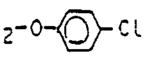 | 63-65 |
| 108 | 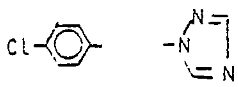 | | CO | $-C(CH_3)_2-CH_2CH_2-O-$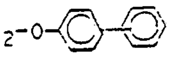 | 113-16 |
| 109 | 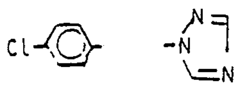 | | CO | $-C(CH_3)_2-CH_2CH_2-O-$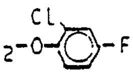 | 1,5581 |
| 110 | 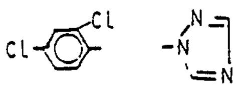 | | CO | $-C(CH_3)_2-CH_2CH_2-O-$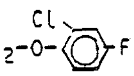 | 79-81 |
| 111 | 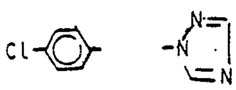 | | CO | 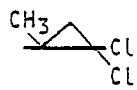 | 1,5555 |
| 112 | 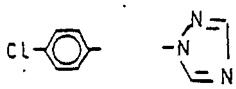 | | CO | 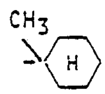 | zähes Oel |
| 113 | 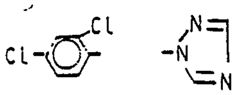 | | CO | 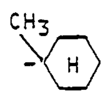 | zähes Oel |
| 114 | 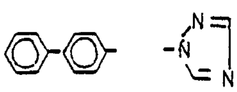 | | CO | 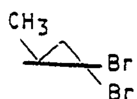 | 56 |
| 115 | 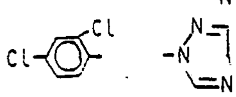 | | CO | 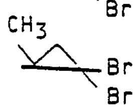 | 136(x$HNO_3$) |
| 116 | 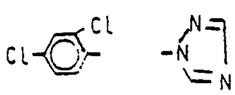 | | CO | 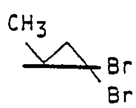 | 125 |
| 117 | 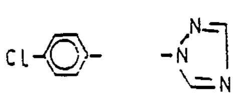 | | CO | 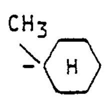 | 1,5412 |
| 118 | 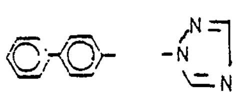 | | CO | 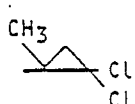 | zähes Oel |
| 119 | 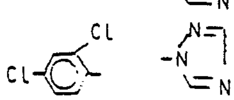 | | CO | 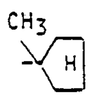 | zähes Oel |
| 120 | 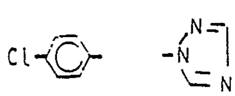 | | CO | 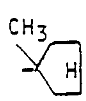 | zähes Oel |

(Fortsetzung)

| Bsp. Nr. | Ar | Az | B | R | Fp(°C) bzw. $n_D^{20}$ |
|---|---|---|---|---|---|
| 121 | 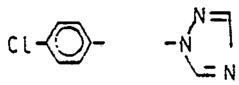 | | CO | 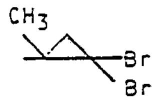 | 1,578 |
| 122 | 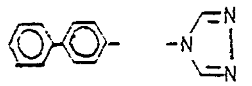 | | CO | $-C(CH_3)_2-CH_2CH_2-O-$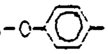$-Cl$ | 179-80 |
| 123 | 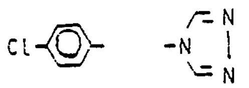 | | CO | $-C(CH_3)_2-CH_2CH_2-O-$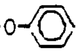 | Harz |
| 124 | 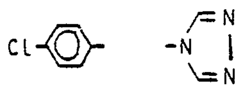 | | CO | $-C(CH_3)_2-CH_2CH_2-O-$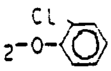$-Cl$ | Harz |
| 125 | 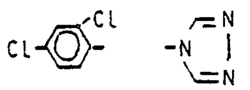 | | CO | $-C(CH_3)_2-CH_2CH_2-O-$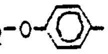$-Cl$ | 121-22 |
| 126 | 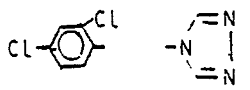 | | CO | $-C(CH_3)_2-CH_2CH_2-O-$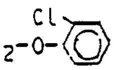$-Cl$ | 110-15 |
| 127 | 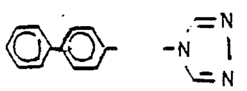 | | CO | $-C(CH_3)_2-CH_2CH_2-O-$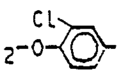$-Cl$ | 157 |
| 128 | 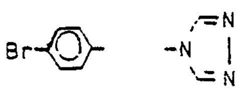 | | CO | $-C(CH_3)_2-CH_2CH_2-O-$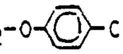$-Cl$ | 146-47 |
| 129 | 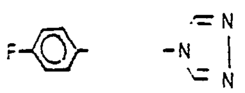 | | CO | $-C(CH_3)_2-CH_2CH_2-O-$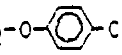$-Cl$ | 122-23 |
| 130 | 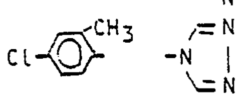 | | CO | $-C(CH_3)_2-CH_2CH_2-O-$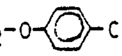$-Cl$ | 142-43 |
| 131 | 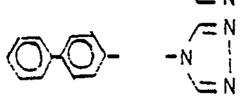 | | CO | $-C(CH_3)_2-CH_2CH_3-O-$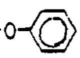 | 154-55 |
| 132 | 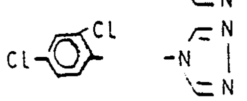 | | CO | $-C(CH_3)_2-CH_2CH_2-O-$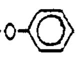 | 110-111 |
| 133 | 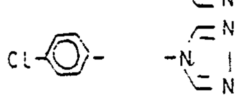 | | CO | $-C(CH_3)_2-CH_2CH_2-O-$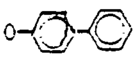 | 149-53 |
| 134 | 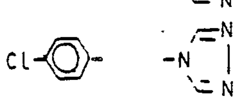 | | CO | $-C(CH_3)_2-CH_2CH_2-O-$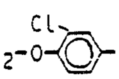$-F$ | 1,5590 |
| 135 | 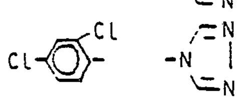 | | CO | $-C(CH_3)_2-CH_2CH_2-O-$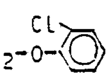$-F$ | Harz |
| 136 | 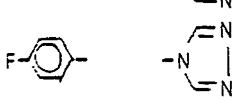 | | CO | $-C(CH_3)_2-C_3H_7-i$ | 122-25 |

(Fortsetzung)

| Bsp. Nr. | Ar | Az | B | R | Fp(°C) bzw. $n_D^{20}$ |
|---|---|---|---|---|---|
| 137 | 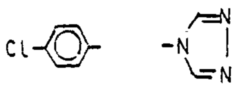 | | CO | 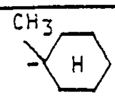 | zähes Oel |
| 138 | 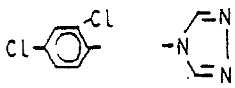 | | CO | 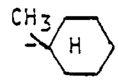 | 110-18 |
| 139 | 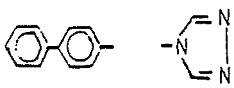 | | CO | $-C(CH_3)_2-C_3H_7-i$ | 224-25 |
| 140 | 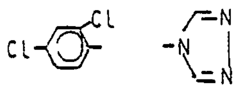 | | CO | $-C(CH_3)_2-C_3H_7-i$ | 148-49 |
| 141 | 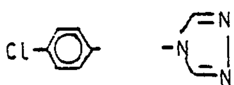 | | CO | $-C(CH_3)_2-C_3H_7-i$ | 143 |
| 142 | 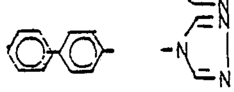 | | CO | $-C(CH_3)_2-CH_2-C(CH_3)_3$ | 206 |
| 143 | 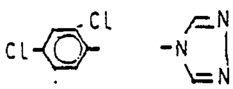 | | CO | $-C(CH_3)_2-CH_2-C(CH_3)_3$ | 138 |
| 144 | 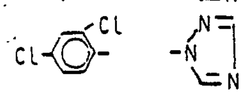 | | CH(OH) | $-C(CH_3)_2-CH_2-$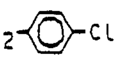 | 144(A-Form) |
| 145 | 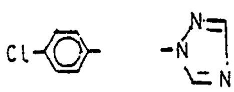 | | CH(OH) | $-C(CH_3)_2-CH_2-$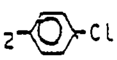 | 35-37 (B-Form) |
| 146 | 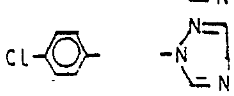 | | CH(OH) | $-C(CH_3)_2-CH_2-$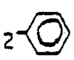 | 157(A-Form) |
| 147 | 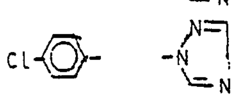 | | CH(OH) | $-C(CH_3)_2-CH_2-$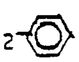 | zähes Oel (B-Form) |
| 148 | 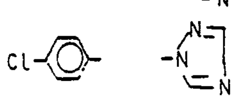 | | CH(OH) | $-C(CH_3)_2-CH_2-$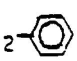 | zähes Oel |
| 149 | 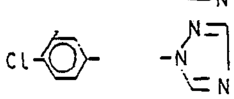 | | CH(OH) | 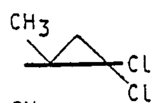 | zähes Oel |
| 150 | 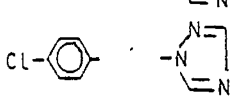 | | CH(OH) | 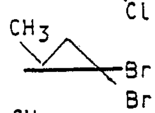 | 168(A-Form) |
| 151 | 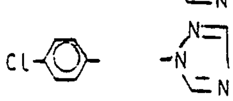 | | CH(OH) | 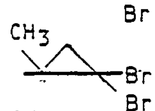 | 116-19 (B-Form) |
| 152 | 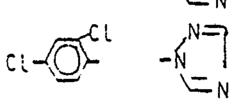 | | CH(OH) | 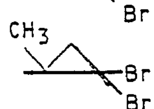 | 145 |
| 153 | 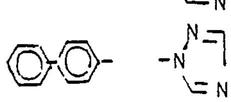 | | CH(OH) | 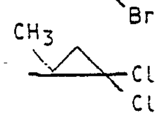 | 80-85 |

(Fortsetzung)

| Bsp. Nr. | Ar | Az | B | R | Fp(°C) bzw. $n_D^{20}$ |
|---|---|---|---|---|---|
| 154 | 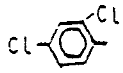 | 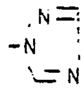 | CH(OH) | 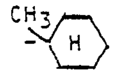 | 84 |
| 155 | 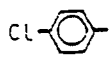 | 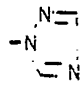 | CH(OH) | 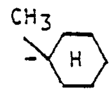 | zähes Oel |
| 156 | 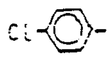 | 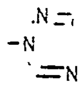 | CO | $-C(CH_3)_2-C_3H_7-n$ | 1,5332 |
| 157 | 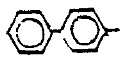 | 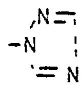 | CH(OH) | $-C(CH_3)_2-C_3H_7-n$ | 110 |
| 158 | 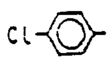 | 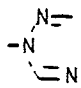 | CH(OH) | $-C(CH_3)_2-C_4H_9-n$ | 1,5310 |
| 159 | 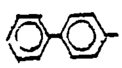 | 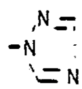 | CH(OH) | $-C(CH_3)_2-C_3H_7-i$ | 103-04 |
| 160 | 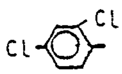 | 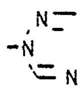 | CH(OH) | $-C(CH_3)_2-C_3H_7-i$ | 113 |
| 161 | 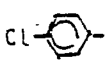 | 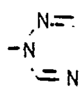 | CH(OH) | $-C(CH_3)_2-C_3H_7-i$ | Harz |
| 162 | 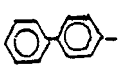 | 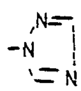 | CH(OH) | $-C(CH_3)_2-CH_2-C(CH_3)_3$ | 120 |
| 163 | 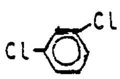 | 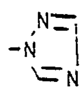 | CH(OH) | $-C(CH_3)_2-CH_2-C(CH_3)_3$ | Harz |
| 164 | 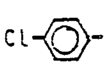 | 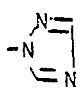 | CH(OH) | $-C(CH_3)_2-$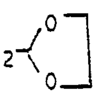 | 120-26 |
| 165 | 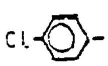 | 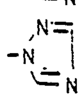 | CH(OH) | $-C(CH_3)_2-CH_2-CH_2-O-$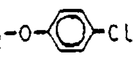 | 155 - 63 |
| 166 | 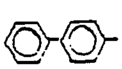 | 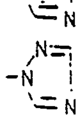 | CH(OH) | $-C(CH_3)_2-CH_2CH_2-O-$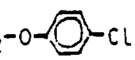 | 81-83 |
| 167 | 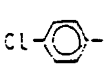 | 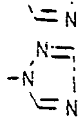 | CH(OH) | $-C(CH_3)_2-CH_2CH_2-O-$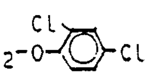 | Harz |
| 168 | 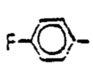 | 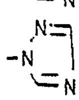 | CH(OH) | $-C(CH_3)_2-CH_2CH_2-O-$ | Harz |
| 169 | 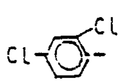 | 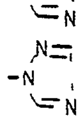 | CH(OH) | $-C(CH_3)_2-CH_2CH_2-O-$ | Harz |
| 170 | 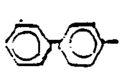 | 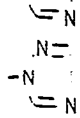 | CH(OH) | $-C(CH_3)_2-CH_2CH_2-O-$ | 118-19 |

(Fortsetzung)

| Bsp. Nr. | Ar | Az | B | R | Fp(°C) bzw. $n_D^{20}$ |
|---|---|---|---|---|---|
| 171 | 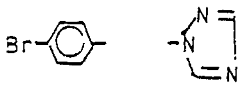 | | CH(OH) | $-C(CH_3)_2-CH_2CH_2$ O- 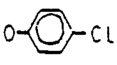 | 115-17 |
| 172 | 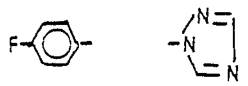 | | CH(OH) | $-C(CH_3)_2-CH_2CH_2-O-$ 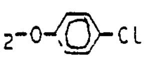 | Harz |
| 173 | 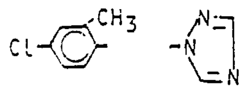 | | CH(OH) | $-C(CH_3)_2-CH_2CH_2-O-$ 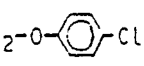 | Harz |
| 174 | 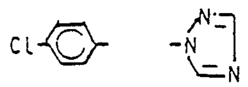 | | CH(OH) | $-C(CH_3)_2-CH_2CH_2-O-$ 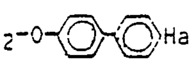 | Harz |
| 175 | 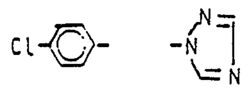 | | CH(OH) | $-C(CH_3)_2-CH_2CH_2-O-$ 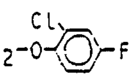 | Harz |
| 176 | 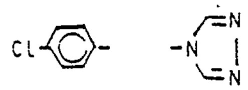 | | CH(OH) | $-C(CH_3)_2-CH_2CH_2-O-$ 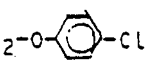 | 104-05 |
| 177 | 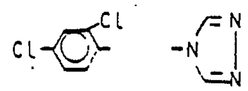 | | CH(OH | $-C(CH_3)_2-CH_2-C(CH_3)_3$ | 128 |
| 178 | 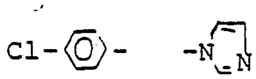 | | CO | $-C(CH_3)_2-CH_2-O-$ 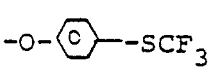 | 1,5490 |
| 179 | 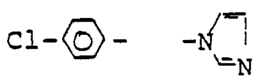 | | CO | $-C(CH_3)_2-CH_2-CH_2-O-$ 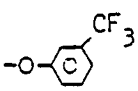 | 1,5378 |
| 180 | 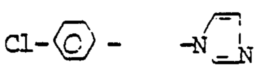 | | CO | $-C(CH_3)_2-CH_2-CH_2-O-$ 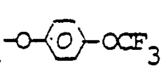 | zähes Öl |
| 181 | 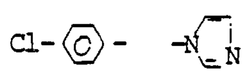 | | CO | $-C(CH_3)_2-CH_2CH_2-O-$ 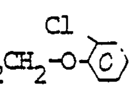 | 1,5686 |
| 182 | 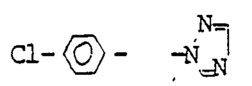 | | CO | $-C(CH_3)_2-CH_2CH_2-O-$ 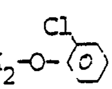 | 1,5689 |
| 183 | 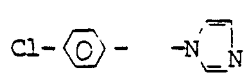 | | CO | $-C(CH_3)_2-CH_2CH_2-O-$ 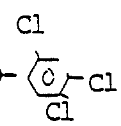 | 1,5738 |
| 184 | 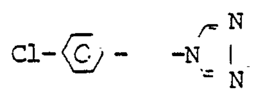 | | CO | $-C(CH_3)_2-CH_2CH_2-O-$ 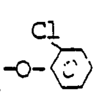 | 126-28 |
| 185 | 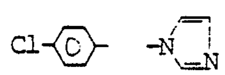 | | CH(OH) | $-C(CH_3)_2-CH_2CH_2-O-$ 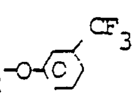 | 1,5291 |
| 186 | 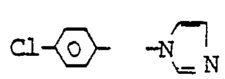 | | CH(OH) | $-C(CH_3)_2-CH_2CH_2-O-$ 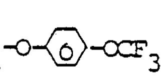 | 134-38 |

(Fortsetzung)

| Bsp. Nr. | Ar | Az | B | R | Fp(°C) bzw. $n_D^{20}$ |
|---|---|---|---|---|---|
| 187 | 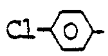 | 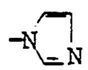 | CO | 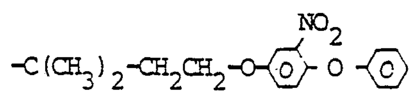 | Harz |
| 188 | 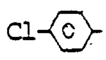 | 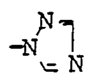 | CO | 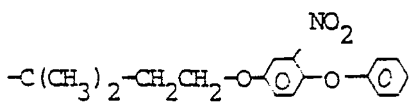 | Harz |
| 189 | 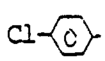 | 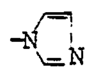 | CO | 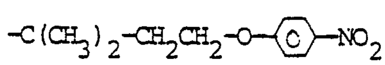 | Harz |
| 190 | 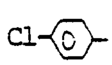 | 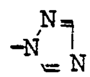 | CO | 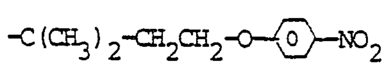 | 99-100 |
| 191 | 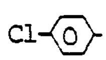 | 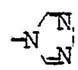 | CO | 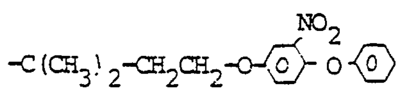 | Harz |
| 192 | 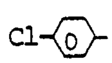 | 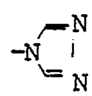 | CO | 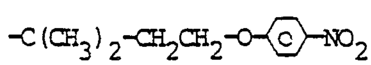 | 135-36 |
| 193 | 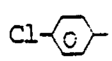 | 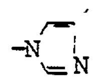 | CH(OH) | 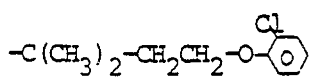 | Harz |
| 194 | 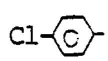 | 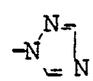 | CH(OH) | 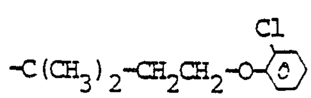 | Harz |
| 195 | 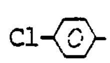 | 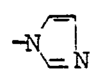 | CH(OH) | 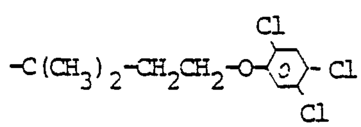 | Harz |
| 196 | 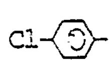 | 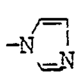 | CH(OH) | 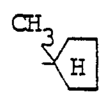 | 123 |
| 197 | 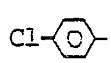 | 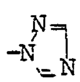 | CH(OH) | 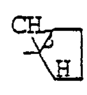 | 104-07 (A-Form) |
| 198 | 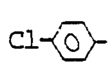 | 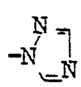 | CH(OH) | 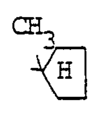 | Öl (B-Form) |
| 199 | 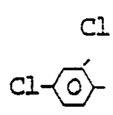 | 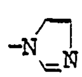 | CH(OH) | 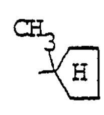 | Öl |

(Fortsetzung)

| Bsp. Nr. | Ar | Az | B | R | Fp (°C) $n_D^{20}$ |
|---|---|---|---|---|---|
| 200 | 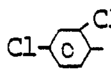 | 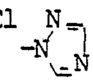 | CH(OH) | 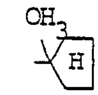 | 94 |
| 201 | 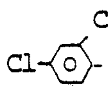 | 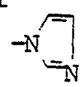 | CO | 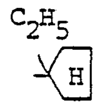 | Öl |
| 202 | 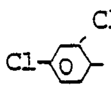 | 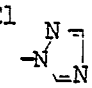 | CO | 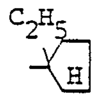 | Öl |
| 203 | 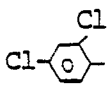 | 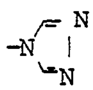 | CO | 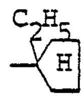 | 175 |
| 204 | 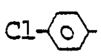 | 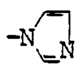 | CO | 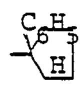 | 91 |
| 205 | 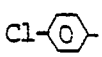 | 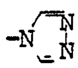 | CO | 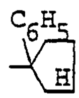 | 156–58 |
| 206 | 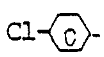 | 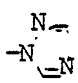 | CO | 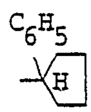 | 107 |
| 207 | 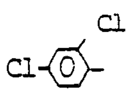 | 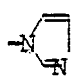 | CH(OH) | 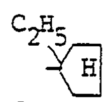 | 107–10 |
| 208 | 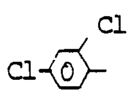 | 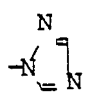 | CH(OH) | 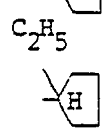 | Öl |
| 209 | 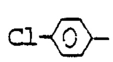 | 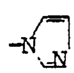 | CH(OH) | 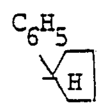 | Öl |
| 210 | 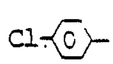 | 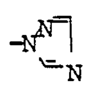 | CH(OH) | 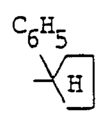 | Öl |
| 211 | 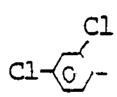 | 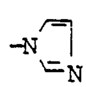 | CO | 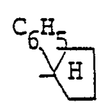 | Öl |
| 212 | 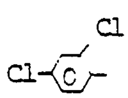 | 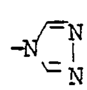 | CO | 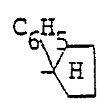 | 138–40 |

(Fortsetzung)

| Bsp. Nr. | Ar | Az | B | R | Fp(°C) $n_D^{20}$ |
|---|---|---|---|---|---|
| 213 | 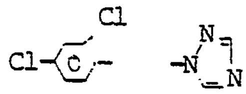 | | CO | 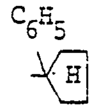 | 102–03 |
| 214 | 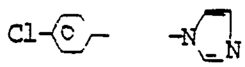 | | CO | 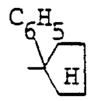 | 98–100 |
| 215 | 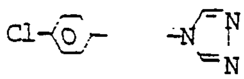 | | CO | 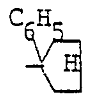 | 160–61 |
| 216 | 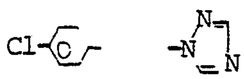 | | CO | 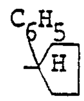 | Öl |
| 217 | 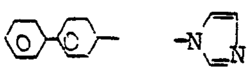 | | CO | 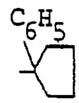 | Öl |
| 218 | 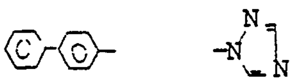 | | CO | 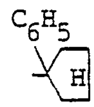 | 103–09 |
| 219 | 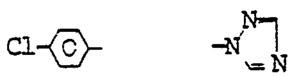 | | CH(OH) | 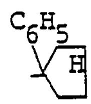 | Öl |
| 220 | 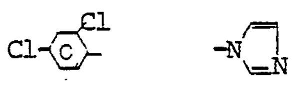 | | CH(OH) | 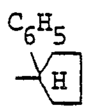 | 111 (A-Form) |
| 221 | 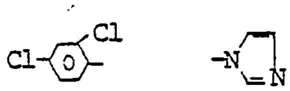 | | CH(OH) | 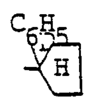 | Öl |
| 222 | 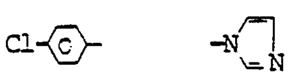 | | CH(OH) | 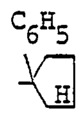 | 114 (A-Form) |
| 223 | 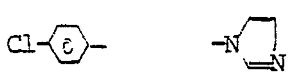 | | CH(OH) | 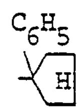 | Öl |
| 224 | 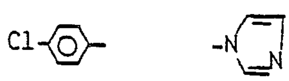 | | CO | $-C(CH_3)_2-CH_2CH_2CH_2Cl$ | 1,5412 |
| 225 | 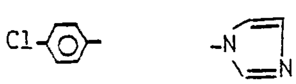 | | CH(OH) | $-C(CH_3)_2-CH_2CH_2-O-$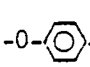$-SCF_3$ | Öl |
| 226 | 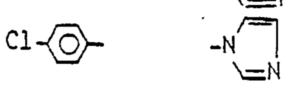 | | CO | 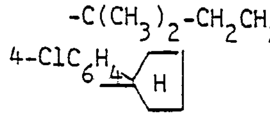 | Öl |
| 227 | 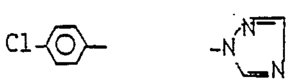 | | CO | 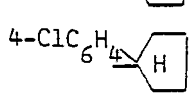 | Öl |

A- und B-Form : die beiden möglichen geometrischen Isomeren

Verwendungsbeispiele

In den nachfolgenden Beispielen werden die nachstehend angegebenen Verbindungen als Vergleichssubstanzen eingesetzt :

(A) 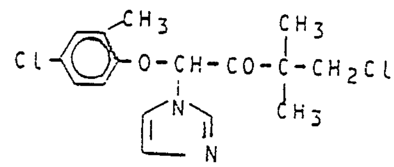

(B) 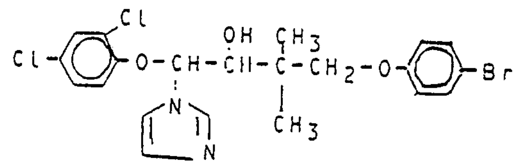

(C) 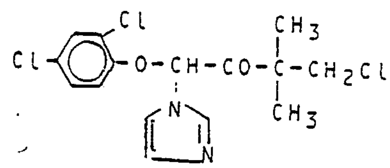

(D) 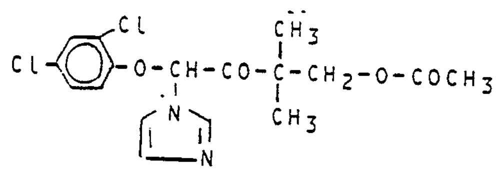

(E) 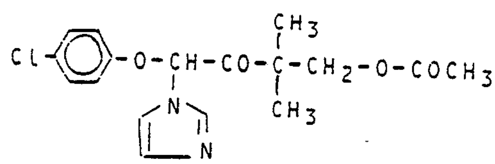

(F) 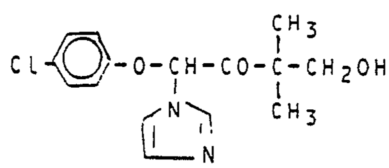

(G) 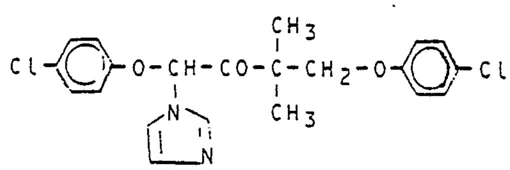

(H) 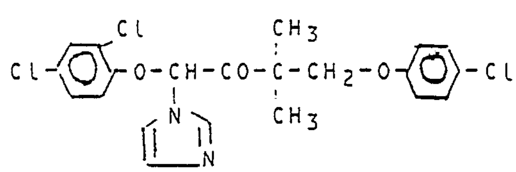

(I) 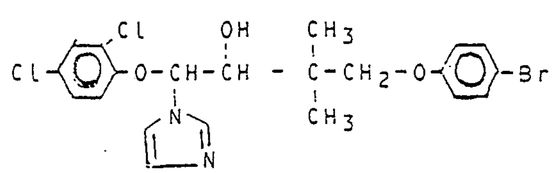

(J) 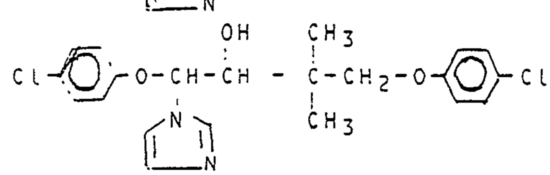

(Fortsetzung)

(K) 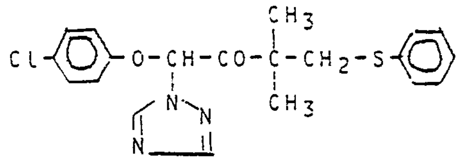

(L) 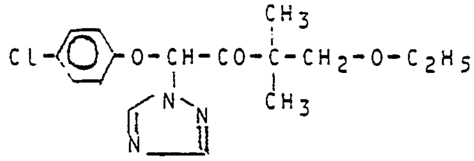

(M) 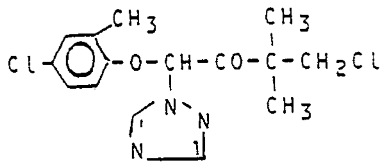

(N) 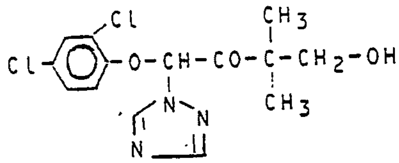

(O) 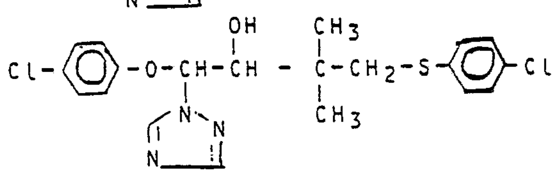

(P) 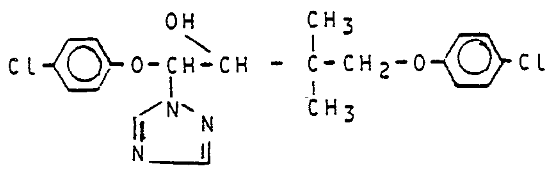

(Q) 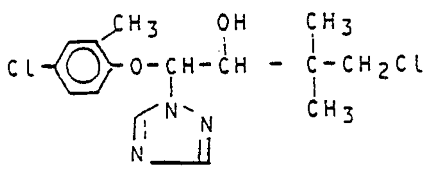

(R) 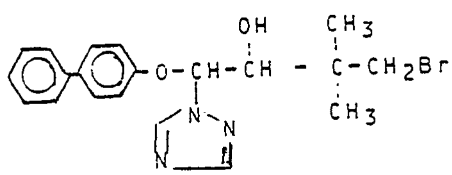

(S) 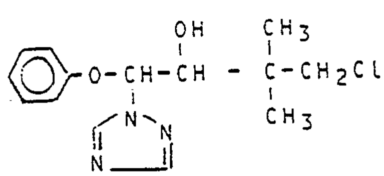

(T) 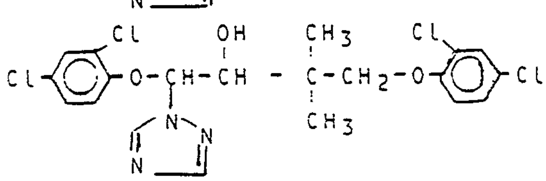

(U) 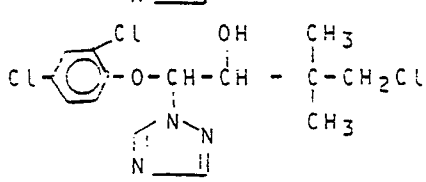

## Beispiel A

Puccinia-Test (Weizen)/protektiv

Lösungsmittel : 100 Gewichsteile Dimethylformamid
Emulgator : 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit einer Sporensuspension von Puccinia recondita in einer 0,1 %igen wäßrigen Agarlösung inokuliert. Nach Antrocknen besprüht man die Pflanzen mit der Wirkstoffzubereitung taufeucht. Die Pflanzen verbleiben 24 Stunden bei 20 °C und 100 % rel. Luftfeuchtigkeit in einer Inkubationskabine.

Die Pflanzen werden in einem Gewächshaus bei einer Temperatur von ca. 20 °C und einer relativen Luftfeuchtigkeit von ca. 80 % aufgestellt, um die Entwicklung von Rostpusteln zu begünstigen.

10 Tage nach der Inokulation erfolgt die Auswertung.

Eine deutliche Überlegenheit in der Wirksamkeit gegenüber dem Stand der Technik zeigen bei diesem Test z. B. die Verbindungen gemäß folgender Herstellungsbeispiele : 13, 12, 22, 16, 17, 33, 34, 4, 36, 24, 38, 39, 40, 41, 42, 43, 5, 44, 45, 145, 147, 169, 171 und 172.

## Beispiel B

Cochliobolus sativus-Test (Gerste)/protektiv

Lösungsmittel : 100 Gewichtsteile Dimethylformamid
Emulgator : 0,25 Gewichtsteile Alkylarylpolyglykolether

Zu Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Cochliobolus sativus besprüht. Die Pflanzen verbleiben 48 Stunden bei 20 °C und 100 % rel. Luftfeuchtigkeit in einer Inkubationskabine.

Die Pflanzen werden in einem Gewächshaus bei einer Temperatur von ca. 20° und einer relativen Luftfeuchtigkeit von ca. 80 % aufgestellt.

7 Tage nach der Inokulation erfolgt die Auswertung.

Eine deutliche Ueberlegeheit in der Wirksamkeit gegenüber dem Stand der Technik zeigen bei diesem Test z. B. die Verbindungen gemäß folgender Herstellungsbeispiele : 13, 12, 22, 17, 45, 68, 58 und 75.

## Beispiel C

Sphaerotheca-Test (Gurke)/protektiv

Lösungsmittel : 4,7 Gewichtsteile Aceton
Emulgator : 0,3 Gewichtsteile Alkyl-arylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Waser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Sphaerotheca fuliginea bestäubt.

Die Pflanzen werden anschließend bei 23 bis 24 °C und bei einer relativen Luftfeuchtigkeit von ca. 75 % im Gewächshaus aufgestellt.

10 Tage nach der Inokulation erfolgt die Auswertung.

Eine deutliche Überlegenheit in der Wirksamkeit gegenüber dem Stand der Technik zeigen in diesem Test z. B. die Verbindungen gemäß folgender Herstellungsbeispiele : 98, 85, 100, 103, 150, 169, 156, 152 und 83.

Beispiel D

Erysiphe-Test (Gerste)/protektiv

Lösungsmittel : 100 Gewichtsteile Dimethylformamid
Emulgator : 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f. sp. hordei bestäubt.

Die Pflanzen werden in einem Gewächshaus bei einer temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z. B. die Verbindungen gemäß folgender Herstellungsbeispiele : 50, 52, 66, 67, 68, 70, 71, 72, 58, 73, 6, 75, 82, 83, 93, 112, 113 und 106.

Beispiel E

Erysiphe-Test (Gerste)/Saatgutbehandlung

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das Saatgut 3 Minuten lang mit dem Beizmittel in einer verschlossenen Glasflasche.

Die Gerste sät man mit 3 × 12 Korn 2 cm tief in eine Standarderde. 7 Tage nach der Aussaat, wenn die jungen Pflanzen ihr erstes Blatt entfaltet haben, werden sie mit Sporen von Erysiphe graminis f. sp. hordei bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z. B. die Verbindungen gemäß folgender Herstellungsbeispiele : 150.

Beispiel F

Pyricularia-Test (Reis)/protektiv

Lösungsmittel : 12,5 Gewichtsteile Aceton
Emulgator : 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25 °C aufgestellt.

4 Tage nach der Inokulation erfolgt die Auswertung des Krankheitsbefalls.

Eine deutliche Überlegenheit in der Wirksamkeit gegenüber dem Stand der Technik zeigen bei diesem Test z. B. die Verbindungen gemäß folgender Herstellungsbeispiele : 156, 169, 152, 24, 43.

Beispiel G

Pellicularia-Test (Reis)

Lösungsmittel : 12,5 Gewichtsteile Aceton
Emulgator : 0,3 Gewichtsteile Alkylarylpolyglykolether

Zur Herstellung einer zweckmäßigen Wirkstoffzubereitung vermischt man 1 Gewichtsteil Wirkstoff

mit der angegebenen Menge Lösungsmittel und verdünnt das Konzentrat mit Wasser und der angegebenen Menge Emulgator auf die gewünschte Konzentration.

Zur Prüfung auf Wirksamkeit werden junge Reispflanzen im 3- bis 4-Blattstadium tropfnaß gespritzt. Die Pflanzen verbleiben bis zum Abtrocknen im Gewächshaus. Anschließend werden die Pflanzen mit Pellicularia sasakii inokuliert und bei 25 °C und 100 % relativer Luftfeuchtigkeit aufgestellt.

5 bis 8 Tage nach der Inokulation erfolgt die Auswertung des Krankheitsbefalles.

Eine deutliche Überlegenheit in der Wirksamkeit gegenüber dem Stand der Technik zeigen bei diesem Test z. B. die Verbindungen gemäß folgender Herstellungsbeispiele : 12, 22, 14, 23, 16, 17, 18, 33, 4, 37, 24, 38, 25, 41, 42, 43 und 44.

## Beispiel H

Agarplattentest

Verwendeter Nährboden
39 Gewichtsteile Kartoffelglykose-Agar
5 Gewichtsteile Agar Agar
10 Gewichtsteile Pepton
5 Gewichtsteile Malz

werden in 1 000 ml destilliertem Wasser gelöst und 30 Minuten bei 121 °C autoklaviert.

Lösungsmittel : 2 Gewichtsteile Wasser-Aceton (97,5 : 2,5)
Mengenverhältnis von Lösungsmittel zu Nährboden : 2 : 100

Zur Herstellung einer zweckmäßigen Wirkstoffzubereitung vermischt man 1 Gewichtsteil Wirkstoff mit der angegebenen Menge Lösungsmittel.

Das Konzentrat wird im genannten Mengenverhältnis mit dem flüssigen Nährboden gründlich vermischt und in Petrischalen gegossen.

Ist der Nährboden erkaltet und fest, werden die Platten mit folgenden Mikroorganismen beimpft und bei ca. 21 °C inkubiert : Cochliobolus miyabeanus.

Die Auswertung erfolgt je nach Wachstumsgeschwindigkeit der Mikroorganismen nach 2 bis 8 Tagen, wobei die Wachstumshemmung als Maß für die Wirkung der Präparate herangezogen wird.

Eine deutliche Überlegenheit gegenüber dem Stand der Technik zeigen in diesem Test die Verbindungen gemäß folgender Herstellungsbeispiele : 145, 147, 156, 157, 167, 168 und 173.

**Patentansprüche**

1. Substituierte Azolylether-ketone und -carbinole der Formel

$$\mathrm{Ar - O - \underset{\displaystyle Az}{\underset{|}{CH}} - B - R} \qquad (I)$$

in welcher

Ar für gegebenenfalls einfach oder mehrfach, gleich oder verschieden substituiertes Phenyl steht, wobei als Substituenten genannt seien : Halogen, Alkyl mit 1 bis 4 Kohlenstoffatomen, Alkoxy und Alkylthio mit jeweils 1 bis 2 Kohlenstoffatomen ; Halogenalkyl, Halogenalkoxy und Halogenalkylthio mit jeweils 1 bis 2 Kohlenstoffatomen und 1 bis 5 gleichen oder verschiedenen Halogenatomen, Alkoxycarbonyl mit 1 bis 4 Kohlenstoffatomen im Alkylteil, Nitro, Cyano oder gegebenenfalls durch Halogen und/oder Alkyl mit 1 bis 2 Kohlenstoffatomen substituiertes Phenyl oder Phenoxy,

Az für 1,2,4-Triazol-1-yl, 1,2,4-Triazol-4-yl oder Imidazol-1-yl steht,

B für die Ketogruppe oder eine CH(OH)-Gruppierung steht und

R für einfach oder mehrfach, gleich oder verschieden substituiertes Cycloalkyl mit 3 bis 7 Kohlenstoffatomen steht, wobei als Substituenten genannt seien : Halogen, Alkyl mit 1 bis 4 Kohlenstoffatomen, Alkoxy mit 1 bis 2 Kohlenstoffatomen und Phenyl ; ferner für die Gruppierungen $—C(CH_3)_2—R^1$, $—C(CH_3)_2—CH_2CH_2—R^2$, $—C(CH_3)_2—(CH_2)_m—R^3$ und $—C(CH_3)_2—(CH_2)_n—R^4$ steht ; wobei

$R^1$ für geradkettiges oder verzweigtes Alkyl mit 2 bis 6 Kohlenstoffatomen, für Alkenyl mit 3 bis 6 Kohlenstoffatomen und Alkinyl mit 2 bis 4 Kohlenstoffatomen steht, ferner für die Aldehydgruppe und deren Derivate steht, wie Oxime, Oximether und Acetale, wie Dialkylacetale mit 1 bis 4 Kohlenstoffatomen in jedem Alkylteil oder für Dioxan und Dioxolan steht,

$R^2$ für gegebenenfalls einfach oder mehrfach, gleich oder verschieden substituiertes Phenoxy steht, wobei als Substituenten die bei Ar oben genannten Phenylsubstituenten infrage kommen ;

$R^3$ für gegebenenfalls einfach oder mehrfach, gleich oder verschieden substituiertes Phenyl steht,

wobei als Substituenten die bei Ar oben genannten Phenylsubstituenten infrage kommen ;

$R^4$ für Fluor, Chlor oder Brom steht ;

m für ganze Zahlen von 1 bis 4 steht und

n für ganze Zahlen von 2 bis 4 steht,

sowie deren Säureadditionssalze und Metallsalzkomplexe.

2. Verbindungen der allgemeinen Formel (I) in Anspruch 1, wobei

Ar für gegebenenfalls einfach bis dreifach, gleich oder verschieden substituiertes Phenyl steht, wobei als Substituenten genannt seien : Fluor, Chlor, Brom, Methyl, Ethyl, Isopropyl, Methoxy, Methylthio, Trifluormethyl, Trifluormethoxy, Trifluormethylthio, Methoxycarbonyl, Ethoxycarbonyl, Nitro, Cyano, Phenyl, Chlorphenyl, Phenoxy,

R für einfach bis dreifach, gleich oder verschieden substituiertes Cycloalkyl mit 3 bis 6 Kohlenstoffatomen steht, wobei als Substituenten genannt seien : Fluor, Chlor, Brom, Methyl, Ethyl, Methoxy, Ethoxy und Phenyl ; ferner für die Gruppierungen $—C(CH_3)_2—R^1$, $—C(CH_3)_2—CH_2CH_2—R^2$, $—C(CH_3)_2—(CH_2)m—R^3$ und $—C(CH_3)_2—(CH_2)n—R^4$ steht ; wobei

$R^1$ für Ethyl, n-Propyl, i-Propyl, n-Butyl, i-Butyl, sek.-Butyl, tert.-Butyl oder Neopentyl steht, weiterhin für Alkenyl mit 3 bis 6 Kohlenstoffatomen und Alkinyl mit 2 bis 4 Kohlenstoffatomen steht, ferner für die Aldehydgruppe und deren Derivate steht, wie Oxime und Oximether, zu letzteren seien genannt : Alkylether mit 1 bis 4 Kohlenstoffatomen, Alkenylether mit 2 bis 4 Kohlenstoffatomen, Alkinylether mit 2 bis 4 Kohlenstoffatomen und gegebenenfalls durch Halogen ein- bis dreifach substituierte Benzylether ; und für Dialkylacetale mit 1 bis 4 Kohlenstoffatomen in jedem Alkylteil stehen, sowie für Dioxan und Dioxolan steht ;

$R^2$ für gegebenenfalls einfach bis dreifach, gleich oder verschieden substituiertes Phenoxy steht, wobei als Substituenten die bei Ar oben genannten Phenylsubstituenten infrage kommen ;

$R^3$ für gegebenenfalls einfach bis dreifach, gleich oder verschieden substituiertes Phenyl steht, wobei als Substituenten die bei Ar oben genannten Phenylsubstituenten infrage kommen ;

$R^4$ für Fluor oder Chlor steht ;

m für 1 oder 2 steht ;

n für 2 steht ;

Az und B die in Anspruch 1 angegebene Bedeutung besitzen.

3. Verfahren zur Herstellung von Substituierten Azolylether-ketonen und -carbinolen der Formel

$$Ar - O - \underset{\substack{| \\ Az}}{CH} - B - R \qquad (I)$$

in welcher

Ar für gegebenenfalls einfach oder mehrfach, gleich oder verschieden substituiertes Phenyl steht, wobei als Substituenten genannt seien : Halogen, Alkyl mit 1 bis 4 Kohlenstoffatomen, Alkoxy und Alkylthio mit jeweils 1 bis 2 Kohlenstoffatomen ; Halogenalkyl, Halogenalkoxy und Halogenalkylthio mit jeweils 1 bis 2 Kohlenstoffatomen und 1 bis 5 gleichen oder verschiedenen Halogenatomen, Alkoxycarbonyl mit 1 bis 4 Kohlenstoffatomen im Alkylteil, Nitro, Cyano oder gegebenenfalls durch Halogen und/oder Alkyl mit 1 bis 2 Kohlenstoffatomen substituiertes Phenyl oder Phenoxy,

Az für 1,2,4-Triazol-1-yl, 1,2,4-Triazol-4-yl oder Imidazol-1-yl steht,

B für die Ketogruppe oder eine CH(OH)-Gruppierung steht und

R für einfach oder mehrfach, gleich oder verschieden substituiertes Cycloalkyl mit 3 bis 7 Kohlenstoffatomen steht, wobei als Substituenten genannt seien : Halogen, Alkyl mit 1 bis 4 Kohlenstoffatomen, Alkoxy mit 1 bis 2 Kohlenstoffatomen und Phenyl ; ferner für die Gruppierungen $—C(CH_3)_2—R^1$, $—C(CH_3)_2—CH_2CH_2—R^2$, $—C(CH_3)_2—(CH_2)_m—R^3$ und $—C(CH_3)_2—(CH_2)_n—R^4$ steht ; wobei

$R^1$ für geradkettiges oder verzweigtes Alkyl mit 2 bis 6 Kohlenstoffatomen, für Alkenyl mit 3 bis 6 Kohlenstoffatomen und Alkinyl mit 2 bis 4 Kohlenstoffatomen steht, ferner für die Aldehydgruppe und deren Derivate steht, wie Oxime, Oximether und Acetale, wie Dialkylacetale mit 1 bis 4 Kohlenstoffatomen in jedem Alkylteil oder für Dioxan und Dioxolan steht ;

$R^2$ für gegebenenfalls einfach oder mehrfach, gleich oder verschieden substituiertes Phenoxy steht, wobei als Substituenten die bei Ar oben genannten Phenylsubstituenten infrage kommen ;

$R^3$ für gegebenenfalls einfach oder mehrfach, gleich oder verschieden substituiertes Phenyl steht, wobei als Substituenten die bei Ar oben genannten Phenylsubstituenten infrage kommen ;

$R^4$ für Fluor, Chlor oder Brom steht ;

m für ganze Zahlen von 1 bis 4 steht und

n für ganze Zahlen von 2 bis 4 steht,

sowie von deren Säureadditions-Salzen und Metallsalz-Komplexen, dadurch gekennzeichnet, daß man

a) Halogenetherketone der Formel

$$Ar - O - \underset{\substack{| \\ Hal}}{CH} - CO - R \qquad (II)$$

in welcher

Ar und R die oben angegebene Bedeutung haben und

Hal für Chlor oder Brom steht,

mit 1,2,4-Triazol oder Imidazol in Gegenwart eines Säurebindemittels und gegebenenfalls in Gegenwart eines Verdünnungsmittels umsetzt ; oder

b) Dihalogenketone der Formel

$$(Hal)_2CH{-}CO{-}R \qquad (III)$$

in welcher Hal und R die oben angegebene Bedeutung haben, mit 1,2,4-Triazol oder Imidazol und mit einem Phenol der Formel

$$Ar{-}OH \qquad (IV)$$

in welcher Ar die oben angegebene Bedeutung hat, in Gegenwart eines Säurebindemittels und in Gegenwart eines Verdünnungsmittels umsetzt ; oder gegebenenfalls

c) die nach den Verfahren (a) oder (b) erhaltenen Azolylether-ketone der Formel

$$Ar - O - \underset{\displaystyle Az}{\underset{|}{CH}} - CO - R \qquad (Ia)$$

in welcher Ar, Az und R die oben angegebene Bedeutung haben, nach bekannten Methoden in üblicher Weise reduziert, und gegebenenfalls zur Herstellung von Säureadditionssalzen und Metallsalzkomplexen an die so erhaltenen Verbindungen der Formel (I) anschließend eine Säure oder ein Metallsalz addiert.

4. Fungizide Mittel, gekennzeichnet durch einen Gehalt an mindestens einem substituierten Azolylether-keton oder -carbinol der Formel (I) in Anspruch 1 bzw. eines Säureadditions-Salzes oder Metallsalz-Komplexes einer Verbindung der Formel (I).

5. Verfahren zur Bekämpfung von Pilzen, dadurch gekennzeichnet, daß man substituierte Azolylether-ketone oder -carbinole der Formel (I) in Anspruch 1 bzw. deren Säureadditions-Salze oder Metallsalz-Komplexe auf Pilze oder ihren Lebensraum einwirken läßt.

6. Verwendung von substituierten Azolylether-ketonen und -carbinolen der Formel (I) in Anspruch 1 bzw. deren Säureadditions-Salze oder Metallsalz-Komplexe zur Bekämpfung von Pilzen.

7. Verwendung von substituierten Azolylether-ketonen und -carbinolen der Formel (I) in Anspruch 1 bzw. deren Säureadditions-Salze oder Metallsalz-Komplexe als Pflanzenschutzmittel.

8. Verfahren zur Herstellung von fungiziden Mitteln, dadurch gekennzeichnet, daß man substituierte Azolylether-ketone und -carbinole der Formel (I) in Anspruch 1 bzw. deren Säureadditions-Salze oder Metallsalz-Komplexe mit Streckmitteln und/oder oberflächenaktiven Mitteln vermischt.

**Claims**

1. Substituted azolylether-ketones and -carbinols of the formula

$$Ar - O - \underset{\displaystyle Az}{\underset{|}{CH}} - B - R \qquad (I)$$

in which

Ar represents phenyl which is optionally monosubstituted or polysubstituted by identical or different substituents, the following being mentioned as substituents : halogen, alkyl having 1 to 4 carbon atoms, alkoxy and alkylthio, each having 1 to 2 carbon atoms ; halogenoalkyl, halogenoalkoxy and halogenoalkylthio, each having 1 to 2 carbon atoms and 1 to 5 identical or different halogenatoms, alkoxycarbonyl having 1 to 4 carbon atoms in the alkyl part, nitro, cyano or phenoxy or phenyl which is optionally substituted by halogen and/or alkyl having 1 to 2 carbon atoms,

Az represents 1,2,4-triazol-1-yl, 1,2,4-triazol-4-yl or imidazol-1-yl,

B represents the keto group or a CH(OH) grouping and

R represents cycloalkyl which has 3 to 7 carbon atoms and is monosubstituted or polysubstituted by identical or different substituents, the following being mentioned as substituents : halogen, alkyl having 1 to 4 carbon atoms, alkoxy having 1 to 2 carbon atoms and phenyl ; and furthermore represents the groupings $-C(CH_3)_2-R^1$, $-C(CH_3)_2-CH_2CH_2-R^2$, $-C(CH_3)_2-(CH_2)_m-R^3$ and $-C(CH_3)_2-(CH_2)_n-R^4$ ; wherein

$R^1$ represents straight-chain or branched alkyl having 2 to 6 carbon atoms, alkenyl having 3 to 6

carbon atoms and alkinyl having 2 to 4 carbon atoms, and also represents the aldehyde group and its derivatives, such as oximes, oxime-ethers and acetals, such as dialkyl acetals having 1 to 4 carbon atoms in each alkyl part, or represents dioxane and dioxolane,

$R^2$ represents phenoxy which is optionally monosubstituted or polysubstituted by identical or different substituents, suitable substituents being the phenyl substituents mentioned above in the case of Ar ;

$R^3$ represents phenyl which is optionally monosubstituted or polysubstituted by identical or different substituents, suitable substituents being the phenyl substituents mentioned above in the case of Ar ;

$R^4$ represents fluorine, chlorine or bromine ;

m represents integers from 1 to 4 and

n represents integers from 2 to 4,

and their acid addition salts and metal salt complexes.

2. Compounds of the general formula (I) in Claim 1, wherein

Ar represents phenyl which is optionally monosubstituted to trisubstituted by identical or different substituents, the following being mentioned as substituents : fluorine, chlorine, bromine, methyl, ethyl, isopropyl, methoxy, methylthio, trifluoromethyl, trifluoromethoxy, trifluoromethylthio, methoxycarbonyl, ethoxycarbonyl, nitro, cyano, phenyl, chlorophenyl or phenoxy,

R represents cycloalkyl which has 3 to 6 carbon atoms and is monosubstituted to trisubstituted by identical or different substituents, the following being mentioned as substituents : fluorine, chlorine, bromine, methyl, ethyl, methoxy, ethoxy and phenyl ; and also represents the groupings $-C(CH_3)_2-R^1$, $-C(CH_3)_2-CH_2CH_2-R^2$, $-C(CH_3)_2-(CH_2)m-R^3$ and $-C(CH_3)_2-(CH_2)n-R^4$ ; wherein

$R^1$ represents ethyl, n-propyl, i-propyl, n-butyl, i-butyl, sec.-butyl, tert.-butyl or neopentyl, and furthermore represents alkenyl having 3 to 6 carbon atoms and alkinyl having 2 to 4 carbon atoms, and also represents the aldehyde group and its derivatives, such as oximes and oxime-ethers, the following being mentioned in connection with the latter : alkyl ethers having 1 to 4 carbon atoms, alkenyl ethers having 2 to 4 carbon atoms, alkinyl ethers having 2 to 4 carbon atoms and benzyl ethers which are optionally monosubstituted to trisubstituted by halogen ; and represent dialkyl acetals having 1 to 4 carbon atoms in each alkyl part, and represents dioxane and dioxolane ;

$R^2$ represents phenoxy which is optionally monosubstituted to trisubstituted by identical or different substituents, suitable substituents being the phenyl substituents mentioned above in the case of Ar ;

$R^3$ represents phenyl which is optionally monosubstituted to trisubstituted by identical or different substituents, suitable substituents being the phenyl substituents mentioned above in the case of Ar ;

$R^4$ represents fluorine or chlorine ;

m represents 1 or 2 ;

n represents 2 ; and

Az and B have the meaning given in Claim 1.

3. Process for the preparation of substituted azolylether-ketones and -carbonols of the formula

$$\begin{array}{c} Ar - O - \underset{\displaystyle Az}{\underset{|}{CH}} - B - R \end{array} \qquad (I)$$

in which

Ar represents phenyl which is optionally monosubstituted or polysubstituted by identical or different substituents, the following being mentioned as substituents : halogen, alkyl having 1 to 4 carbon atoms, alkoxy and alkylthio, each having 1 to 2 carbon atoms ; halogenoalkyl, halogenoalkoxy and halogenoalkylthio, each having 1 to 2 carbon atoms and 1 to 5 identical or different halogen atoms, alkoxycarbonyl having 1 to 4 carbon atoms in the alkyl part, nitro, cyano or phenoxy or phenyl which is optionally substituted by halogen and/or alkyl having 1 to 2 carbon atoms,

Az represents 1,2,4-triazol-1-yl, 1,2,4-triazol-4-yl or imidazol-1-yl,

B represents the keto group or a CH(OH) grouping and

R represents cycloalkyl which has to 3 to 7 carbon atoms and is monosubstituted or polysubstituted by identical or different substituents, the following being mentioned as substituents : halogen, alkyl having 1 to 4 carbon atoms, alkoxy having 1 to 2 carbon atoms and phenyl ; and furthermore represents the groupings $-C(CH_3)_2-R^1$, $-C(CH_3)_2-CH_2CH_2-R^2$, $-C(CH_3)_2-(CH_2)_m-R^3$ and $-C(CH_3)_2-(CH_2)_n-R^4$ ; wherein

$R^1$ represents straight-chain or branched alkyl having 2 to 6 carbon atoms, alkenyl having 3 to 6 carbon atoms and alkinyl having 2 to 4 carbon atoms, and also represents the aldehyde group and its derivatives, such as oximes, oxime-ethers and acetals, such as dialkyl acetals having 1 to 4 carbon atoms in each alkyl part, or represents dioxane and dioxolane,

$R^2$ represents phenoxy which is optionally monosubstituted or polysubstituted by identical or different substituents, suitable substituents being the phenyl substituents mentioned above in the case of Ar ;

$R_3$ represents phenyl which is optionally monosubstituted or polysubstituted by identical or different substituents, suitable substituents being the phenyl substituents mentioned above in the case of Ar ;

$R^4$ represents fluorine, chlorine or bromine ;
m represents integers from 1 to 4 and
n represents integers from 2 to 4,

and their acid addition salts and metal salt complexes, characterised in that

a) halogenoether-ketones of the formula

$$Ar - O - \underset{\underset{Hal}{|}}{CH} - CO - R \qquad (II)$$

in which

Ar and R have the meaning given above and
Hal represents chlorine or bromine,

are reacted with 1,2,4-triazole or imidazole in the presence of an acid-binding agent and, if appropriate, in the presence of a diluent ; or

b) dihalogenoketones of the formula

$$(Hal)_2CH—CO—R \qquad (III)$$

in which Hal and R have the meaning given above, are reacted with 1,2,4-triazole or imidazole and with a phenol of the formula

$$Ar—OH \qquad (IV)$$

in which Ar has the meaning given above, in the presence of an acid-binding agent and in the presence of a diluent ; or, if appropriate,

c) the azolylether-ketones obtained by process (a) or (b), of the formula

$$Ar - O - \underset{\underset{Az}{|}}{CH} - CO - R \qquad (Ia)$$

in which Ar, Az and R have the meaning given above, are reduced in a customary manner by known methods, and, if desired, for the preparation of acid addition salts and metal salt complexes ; an acid or a metal salt is then added on to the compounds of the formula (I) thus obtained.

4. Fungicidal agents, characterised in that they contain at least one substituted azolylethyl-ketone or -carbinol of the formula (I) in Claim 1 or an acid addition salt or metal salt complex of a compound of the formula (I).

5. Method of combating fungi, characterised in that substituted azolylether-ketones or -carbinols of the formula (I) in Claim 1 or their acid addition salts or metal salt complexes are allowed to act on fungi or their habitat.

6. Use of substituted azolylether-ketones and -carbinols of the formula (I) in Claim 1 or their acid addition salts or metal salt complexes for combating fungi.

7. Use of substituted azolylether-ketones and -carbinols of the formula (I) in Claim 1 or their acid addition salts or metal salt complexes as plant protection agents.

8. Process for the preparation of fungicidal agents, characterised in that substituted azolylether-ketones and -carbinols of the formula (I) in Claim 1 or their acid addition salts or metal salt complexes are mixed with extenders and/or surface-active agents.

**Revendications**

1. Azolyléthercétones et -carbinols substitués de formule

$$Ar - O - \underset{\underset{Az}{|}}{CH} - B - R \qquad (I)$$

dans laquelle

Ar désigne un groupe phényle portant éventuellement un ou plusieurs substituants identiques ou différents, ces substituants pouvant être : un halogène, un groupe alkyle ayant 1 à 4 atomes de carbone, un groupe alkoxy et un groupe alkylthio ayant chacun 1 ou 2 atomes de carbone ; un groupe halogénalkyle, un groupe halogénalkoxy et un groupe halogénalkylthio ayant chacun 1 ou 2 atomes de carbone et 1 à 5 atomes d'halogènes identiques ou différents, un groupe alkoxycarbonyle ayant 1 à 4

atomes de carbone dans la partie alkyle, un groupe nitro, cyano ou un groupe phényle ou phénoxy éventuellement substitué par un halogène et/ou un radical alkyle ayant 1 ou 2 atomes de carbone.

Az est un groupe 1,2,4-triazole-1-yle, 1,2,4-triazolyle-4-yle ou imidazole-1-yle,

B est le groupe céto ou un groupement CH(OH) et

R est un groupe cycloalkyle de 3 à 7 atomes de carbone portant un ou plusieurs substituants identiques ou différents, ces substituants pouvant être : un halogène, un radical alkyle ayant 1 à 4 atomes de carbone, alkoxy ayant 1 ou 2 atomes de carbone et phényle ; en outre les groupements $-C(CH_3)_2-R^1$, $-C(CH_3)_2-CH_2CH_2-R^2$, $-C(CH_3)_2-(CH_2)_m-R^3$ et $-C(CH_3)_2-(CH_2)_n-R^4$ ; dans lesquels

$R^1$ est un groupe alkyle à chaîne droite ou à chaîne ramifiée ayant 2 à 6 atomes de carbone, un groupe alcényle ayant 3 à 6 atomes de carbone et un groupe alcynyle ayant 2 à 4 atomes de carbone, en outre le groupe aldéhyde et ses dérivés, tels qu'oximes, éthers d'oximes et acétals, tels que dialkylacétals ayant 1 à 4 atomes de carbone dans chaque partie alkyle ou le dioxanne et le dioxolane,

$R^2$ est un groupe phénoxy portant éventuellement un ou plusieurs substituants identiques ou différents, ces substituants pouvant être ceux qui sont considérés à propos des substituants du groupe phényle mentionnés ci-dessus pour Ar ;

$R^3$ est un groupe phényle portant éventuellement un ou plusieurs substituants identiques ou différents, ces substituants pouvant être les substituants du groupe phényle mentionnés ci-dessus pour Ar,

$R^4$ désigne le fluor, le chlore ou le brome ;

m représente des nombres entiers de 1 à 4 et

n représente des nombres entiers de 2 à 4,

ainsi que leurs sels d'addition d'acides et leurs complexes de sels métalliques.

2. Composés de formule générale (I) suivant la revendication 1, dans lesquels

Ar désigne un groupe phényle portant éventuellement un ou plusieurs substituants identiques ou différents, ces substituants pouvant être : le fluor, le chlore, le brome, le groupe méthyle, éthyle, isopropyle, méthoxy, méthylthio, trifluorométhyle, trifluorométhoxy, trifluorométhylthio, méthoxycarbonyle, éthoxycarbonyle, nitro, cyano, phényle, chlorophényle, phénoxy,

R est un groupe cycloalkyle de 3 à 6 atomes de carbone portant un à trois substituants identiques ou différents, ces substituants pouvant être : le fluor, le chlore, le brome, les groupes méthyle, éthyle, méthoxy, éthoxy et phényle ; en outre les groupements $-C(CH_3)_2-R^1$, $-C(CH_3)_2-CH_2CH_2-R^2$, $-C(CH_3)_2-(CH_2)_m-R^3$ et $-C(CH_3)_2-(CH_2)_n-R^4$ ; dans lesquels

$R^1$ est un groupe éthyle, n-propyle, isopropyle, n-butyle, isobutyle, sec.-butyle, tertiobutyle ou néopentyle, en outre un groupe alcényle ayant 3 à 6 atomes de carbone et un groupe alcynyle ayant 2 à 4 atomes de carbone, en outre le groupe aldéhyde et ses dérivés tels qu'oximes et éthers d'oximes, parmi lesquels on peut mentionner : des éthers d'alkyle ayant 1 à 4 atomes de carbone, des éthers d'alcényle ayant 2 à 4 atomes de carbone, des éthers d'alcynyle ayant 2 à 4 atomes de carbone et des éthers de benzyle éventuellement substitués une à trois fois par un halogène ; et des dialkylacétals ayant 1 à 4 atomes de carbone dans chaque partie alkyle, ainsi que le dioxanne et le dioxolane ;

$R^2$ est un groupe phénoxy portant éventuellement un à trois substituants identiques ou différents, et on considère alors comme substituants les substituants du groupe phényle mentionnés ci-dessus pour Ar ;

$R^3$ est un groupe phényle portant éventuellement un à trois substituants identiques ou différents, et on considère alors comme substituants les substituants du groupe phényle mentionnés ci-dessus pour Ar ;

$R^4$ désigne le fluor ou le chlore ;

m a la valeur 1 ou 2 ;

n a la valeur 2 ;

Az et B possèdent la définition indiquée dans la revendication 1 ;

3. Procédé de production d'azolyléther-cétones et -carbinols substitués de formule

$$Ar - O - \underset{\substack{| \\ Az}}{CH} - B - R \qquad (I)$$

dans laquelle

Ar désigne un groupe phényle portant éventuellement un ou plusieurs substituants identiques ou différents, ces substituants pouvant être : un halogène, un groupe alkyle ayant 1 à 4 atomes de carbone, un groupe alkoxy et un groupe alkylthio ayant chacun 1 ou 2 atomes de carbone ; un groupe halogénalkyle, un groupe halogénalkoxy et un groupe halogénalkylthio ayant chacun 1 ou 2 atomes de carbone et 1 à 5 atomes d'halogènes identiques ou différents, un groupe alkoxycarbonyle ayant 1 à 4 atomes de carbone dans la partie alkyle, un groupe nitro, cyano ou un groupe phényle ou phénoxy éventuellement substitué par un halogène et/ou un radical alkyle ayant 1 ou 2 atomes de carbone,

Az est un groupe 1,2,4-triazole-1-yle, 1,2,4-triazolyle-4-yle ou imidazole-1-yle,

B est le groupe céto ou un groupement CH(OH) et

R est un groupe cycloalkyle de 3 à 7 atomes de carbone portant un ou plusieurs substituants identiques ou différents, ces substituants pouvant être : un halogène, un radical alkyle ayant 1 à 4 atomes de carbone, alkoxy ayant 1 ou 2 atomes de carbone et phényle ; en outre les groupements $-C(CH_3)_2-R^1$, $-C(CH_3)_2-CH_2CH_2-R^2$, $-C(CH_3)_2-(CH_2)_m-R^3$ et $-C(CH_3)_2-(CH_2)_n-R^4$ ; dans lesquels

$R^1$ est un groupe alkyle à chaîne droite ou à chaîne ramifiée ayant 2 à 6 atomes de carbone, un groupe alcényle ayant 3 à 6 atomes de carbone et un groupe alcynyle ayant 2 à 4 atomes de carbone, en outre le groupe aldéhyde et ses dérivés, tels qu'oximes, éthers d'oximes et acétals, tels que dialkylacétals ayant 1 à 4 atomes de carbone dans chaque partie alkyle ou le dioxanne et le dioxolane,

$R^2$ est un groupe phénoxy portant éventuellement un ou plusieurs substituants identiques ou différents, ces substituants pouvant être ceux qui sont considérés à propos des substituants du groupe phényle mentionnés ci-dessus pour Ar ;

$R^3$ est un groupe phényle portant éventuellement un ou plusieurs substituants identiques ou différents, ces substituants pouvant être les substituants du groupe phényle mentionnés ci-dessus pour Ar,

$R^4$ désigne le fluor, le chlore ou le brome ;

m représente des nombres entiers de 1 à 4 et

n représente des nombres entiers de 2 à 4,

ainsi que de leurs sels d'addition d'acides et de leurs complexes de sels métalliques, caractérisé en ce que :

a) on fait réagir des halogénéther-cétones de formule

$$\begin{array}{c} Ar - O - \underset{\mid}{CH} - CO - R \\ \quad\quad\quad Hal \end{array} \quad (II)$$

dans laquelle

Ar et R ont la définition indiquée ci-dessus, et

Hal désigne le chlore ou le brome,

avec le 1,2,4-triazole ou l'imidazole en présence d'un accepteur d'acide et, le cas échéant, en présence d'un diluant ; ou bien

b) on fait réagir des dihalogénocétones de formule

$$(Hal)_2CH-CO-R \quad (III)$$

dans laquelle Hal et R ont la définition indiquée ci-dessus, avec le 1,2,4-triazole ou l'imidazole et avec un phénol de formule

$$Ar-OH \quad (IV)$$

dans laquelle Ar a la définition indiquée ci-dessus, en présence d'un accepteur d'acide et en présence d'un diluant ; ou bien, le cas échéant

c) on réduit de manière classique par des procédés connus les azolyléther-cétones, obtenues selon les procédés (a) ou (b), de formule

$$\begin{array}{c} Ar - O - \underset{\mid}{CH} - CO - R \\ \quad\quad\quad Az \end{array} \quad (Ia)$$

dans laquelle Ar, Az et R ont la définition indiquée ci-dessus, et, le cas échéant, en vue de l'obtention de sels d'addition d'acides et de complexes de sels métalliques, on additionne ensuite un acide ou un sel métallique sur les composés de formule (I) ainsi obtenus.

4. Compositions fongicides, caractérisées par une teneur en au moins une azolyléther-cétone ou un azolyléther-carbinol substitué de formule (I) suivant la revendication 1 ou un sel d'addition d'acide ou un complexe de sel métallique d'un composé de formule (I).

5. Procédé pour combattre des champignons, caractérisé en ce qu'on fait réagir sur des champignons ou sur leur milieu des azolyléther-cétones ou -carbinols substitués de formule (I) suivant la revendication 1 ou leurs sels d'addition d'acides ou leurs complexes de sels métalliques.

6. Utilisation d'azolyléther-cétones et -carbinols substitués de formule (I) suivant la revendication 1 ou de leurs sels d'addition d'acides ou de leurs complexes de sels métalliques pour combattre des champignons.

7. Utilisation d'azolyléther-cétones et -carbinols substitués de formule (I) suivant la revendication 1 ou de leurs sels d'addition d'acides ou de leurs complexes de sels métalliques comme agents phytosanitaires.

8. Procédé de préparation de compositions fongicides, caractérisé en ce qu'on mélange avec des diluants et/ou des agents tensio-actifs, des azolyléther-cétones et -carbinols substitués de formule (I) suivant la revendication 1 ou leurs sels d'addition d'acides ou leurs complexes de sels métalliques.